Europäisches Patentamt

(19) European Patent Office

Office européen des brevets

(11) **EP 0 681 447 B1**

(12) **FASCICULE DE BREVET EUROPEEN**

(45) Date de publication et mention
de la délivrance du brevet:
**30.06.1999 Bulletin 1999/26**

(21) Numéro de dépôt: **94905755.8**

(22) Date de dépôt: **28.01.1994**

(51) Int. Cl.$^6$: **A61B 5/021**, A61B 5/024

(86) Numéro de dépôt international:
**PCT/FR94/00110**

(87) Numéro de publication internationale:
**WO 94/16610 (04.08.1994 Gazette 1994/18)**

(54) **DISPOSITIF DE DETERMINATION D'INFORMATIONS PHYSIOLOGIQUES, ET UTILISATION CORRESPONDANTE**

VORRICHTUNG ZUR BESTIMMUNG VON PHYSIOLOGISCHEN DATEN SOWIE VERWENDUNG DER VORRICHTUNG

DEVICE FOR THE DETERMINATION OF PHYSIOLOGICAL INFORMATION AND CORRESPONDING USE

(84) Etats contractants désignés:
**BE DE FR GB NL SE**

(30) Priorité: **28.01.1993 FR 9301132**

(43) Date de publication de la demande:
**15.11.1995 Bulletin 1995/46**

(73) Titulaires:
• **UNIVERSITE DE RENNES I**
**F-35000 Rennes (FR)**
Etats contractants désignés:
**FR**
• **CENTRE STEPHANOIS DE RECHERCHES MECANIQUES HYDROMECANIQUE ET FROTTEMENT**
**Société anonyme**
**F-42166 Andrezieux Boutheon Cédex (FR)**
Etats contractants désignés:
**BE DE GB NL SE**

(72) Inventeurs:
• **BILLON, Michel**
**F-22300 Lannion (FR)**
• **MARTIN, Eric**
**F-22300 Saint-Michel-en-Grève (FR)**
• **INGREMEAU, Olivier**
**F-22300 Lannion (FR)**
• **PANNETIER, Christophe**
**F-22300 Lannion (FR)**

(74) Mandataire: **Vidon, Patrice et al**
**Cabinet Patrice Vidon,**
**Immeuble Germanium,**
**80, Avenue des Buttes-de-Coesmes**
**35700 Rennes (FR)**

(56) Documents cités:
| | |
|---|---|
| **EP-A- 0 289 700** | **EP-A- 0 448 438** |
| **WO-A-88/02237** | **WO-A-89/05116** |
| **US-A- 4 306 567** | **US-A- 4 409 983** |
| **US-A- 4 860 759** | **US-A- 4 924 871** |

## Description

Le domaine de l'invention est celui de la détermination d'une ou plusieurs informations physiologiques représentatives de l'état d'un sujet humain ou animal. Plus précisément, l'invention concerne un dispositif autonome, au moins en ce qui concerne le relevé de mesures, et capable de fournir des informations physiologiques en continu.

Un domaine particulier de l'invention est celui de l'analyse du sommeil, et notamment de la reconnaissance et de la quantification des différentes phases du sommeil chez un sujet. Dans ce cas, l'invention concerne un dispositif autonome et portatif de reconnaissance de phases de sommeil.

Les applications de la reconnaissance et de la quantification des phases de sommeil sont nombreuses. Elles vont du domaine médical (aide au diagnostic de l'apnée du sommeil en pneumologie, des troubles du sommeil, etc...) au domaine "grand public" (évaluation de la qualité et de la quantité du sommeil d'une personne, réveil dans une phase présélectionnée).

Dans le domaine médical, la reconnaissance des phases du sommeil est généralement obtenue a l'aide de polysomnographes, systèmes complexes et coûteux dont l'utilisation reste réservée à des services hospitaliers spécialisés. Ces systèmes analysent en temps différé une pluralité de signaux biologiques correspondant à une nuit de sommeil. Ils peuvent atteindre un score de bonne reconnaissance des phases de l'ordre de 90%.

Cependant, la technologie d'acquisition des signaux biologiques dans ces systèmes est complexe et peu confortable pour le patient. Elle repose en effet sur l'utilisation d'une série d'électrodes fixées sur le corps du patient, et relié à une unité de traitement. La seule présence de ces électrodes et de leurs câbles de liaison peuvent perturber le sommeil du patient, en particulier s'il s'agit d'une personne souffrant de troubles du sommeil. Cela introduit donc un biais dans les mesures effectuées. Ce biais est généralement encore aggravé par le fait que le patient se trouve dans un environnement particulier (chambre d'hôpital) qui n'est pas le sien habituellement.

Dans le domaine grand public, il n'existe actuellement pas de dispositif abordable et confortable pour le suivi régulier des phases de sommeil.

La reconnaissance des phases du sommeil peut également permettre de contrôler le réveil d'un sujet, en fonction de ces phases. On sait en effet que le réveil doit avoir lieu, pour être agréable, au cours d'une période de sommeil lent léger. On peut aussi souhaiter que le réveil s'effectue dans une phase de sommeil paradoxal, pour se remémoriser ses rêves, ou encore après une ou deux périodes de sommeil profond, qui est le plus réparateur.

Des dispositifs connus ayant cet objectif sont par exemple décrits dans le brevet FR-A-2 597 995 et le certificat d'addition FR-A-2 643 913. Le dispositif du premier document n'est pas efficace, car il se base sur un simple calcul de cycles de sommeil, sans faire intervenir aucune donnée physiologique propre au sujet. Le second document présente un dispositif limité à la reconnaissance du sommeil paradoxal, et semble particulièrement inconfortable, puisqu'il impose la présence d'électrodes à la commissure des paupières.

Un autre dispositif de réveil est décrit dans la demande de brevet FR-A-2 679 453 au nom des mêmes déposants, non encore publiée à la date de dépôt de la présente demande. Ce dispositif repose sur l'utilisation, dans un boîtier portatif, d'un capteur d'activité cardiaque permettant de suivre le pouls du sujet. Sachant que le rythme cardiaque varie en fonction des différentes phases du sommeil, leur reconnaissance est alors, en principe, aisée.

Dans la pratique, les inventeurs ont constaté que la prise en compte de l'information de rythme cardiaque seule ne permet pas d'obtenir des résultats satisfaisants. Le taux de réussite de la reconnaissance par ce système reste en effet inférieur à 70%, ce qui est insuffisant pour en envisager l'utilisation, en particulier dans le domaine médical.

Plus généralement, la connaissance de données physiologiques présente de nombreux avantages, que ce soit pour l'aide au diagnostic en milieu médical, le suivi à long terme de patients, la détection de situations anormales,...

Dans la pratique, les dispositifs fiables offrant ces possibilités sont réservés au domaine médical. Il nécessite la présence d'une machine dédiée reliée au patient par une pluralité de capteurs, avec tous les inconvénients déjà décrits plus haut.

Les quelques dispositifs connus destinés au grand public se limite à la détection du pouls. Il s'agit certes d'une information importante, mais elle en général insuffisante pour tirer une conclusion fiable. On connaît aussi des dispositifs capables de déterminer la tension ou la pression artérielle, mais il s'agit de systèmes complexes nécessitant une manipulation peu aisée et ne pouvant pas délivrer des mesures en continu, mais uniquement à la demande (de même que les sismographes classiques).

Le brevet US-A-4 924 871 présente par exemple un capteur de pression artérielle, comprenant une pluralité de capteurs de pression qui délivrent des signaux qui sont numérisés puis analysés dans un dispositif externe. Pour obtenir des mesures correctes, le dispositif comprend une chambre pressurisable reliée à des moyens pneumatiques externes par une liaison qui contient également les liaisons électriques nécessaires.

L'invention a notamment pour objectif de pallier ces inconvénients de l'état de la technique.

Plus précisément, un objectif de l'invention est de fournir un dispositif de détermination d'informations physiologiques, qui soit ambulatoire, c'est-à-dire autonome et portatif ou, plus précisément, qui n'entrave pas les mouvements et les déplacements du porteur. En particulier, l'invention a pour objectif de fournir un tel dispositif qui ne nécessite ni électrodes sur le corps du sujet, ni liaisons filaires avec un système externe.

Un autre objectif de l'invention est de fournir un tel dispositif, qui puisse être mis en oeuvre en tout lieu, et en particulier dans l'environnement habituel du sujet porteur. L'invention a également pour objectif de fournir un tel dispositif qui puisse être utilisé sur de longues périodes (plusieurs jours), voire en permanence.

Un objectif important de l'invention est de fournir un tel dispositif déterminant une ou plusieurs données physiologiques en continu, et non pas à intervalles très espacés, ou à la demande.

Un objectif particulier de l'invention est de fournir un tel dispositif capable d'assurer la reconnaissance de phases du sommeil, et plus généralement de toutes les informations liées au sommeil ou la somnolence.

Dans ce cas, l'invention a notamment pour objectif de fournir un tel dispositif, qui offre une fiabilité suffisante pour pouvoir être utilisé dans le domaine médical. Pour de telles applications, un taux de réussite de reconnaissance d'au moins 80%, et préférentiellement de 85%, est souhaité.

Un autre objectif de l'invention est de fournir un tel dispositif présentant un coût de revient faible, en particulier par rapport aux polysomnographes. Un objectif particulier de l'invention est encore de fournir un tel dispositif, pouvant être réalisé aisément industriellement, et ne mettant en oeuvre que des moyens connus, courants et peu coûteux.

L'invention a également pour objectif de fournir un tel dispositif, qui soit simple d'usage, et qui, en particulier, ne nécessite aucune connaissance médicale ou technique particulière.

Un autre objectif de l'invention est de fournir un tel dispositif capable de relever plusieurs données physiologiques simultanément, et notamment des données jamais encore relevées par de tels dispositifs.

En conséquence, l'invention a encore pour objectif de fournir un tel dispositif, permettant notamment de reconnaître et quantifier les phases de sommeil, mais également d'assurer un nombre important d'applications nouvelles, dans de nombreux domaines médicaux et grand public.

Ces objectifs, ainsi que d'autres qui apparaîtront par la suite, sont atteints selon l'invention à l'aide d'un dispositif détermination d'informations physiologiques comprenant :

- un premier capteur adapté pour être placé en contact avec la peau d'un sujet et réagissant aux variations de la pression sanguine instantanée dudit sujet, délivrant un signal représentatif de ladite pression sanguine instantanée,
- un second capteur isolé de la peau dudit sujet et réagissant aux mouvements dudit sujet, délivrant un signal représentatif desdits mouvements,

- des moyens de réduction des perturbations présentes dans ledit signal représentatif de la pression sanguine instantanée dues auxdits mouvements, en fonction dudit signal représentatif desdits mouvements, lesdits moyens de réduction délivrant un signal corrigé, et
- des moyens de traitement et d'analyse dudit signal corrigé, délivrant au moins une des informations appartenant au groupe comprenant :

  - une information représentative de la tension artérielle relative dudit sujet et/ou de son évolution dans le temps ;
  - une information représentative de la fréquence respiratoire dudit sujet et/ou de son évolution dans le temps ;
  - une information représentative de l'amplitude respiratoire dudit sujet et/ou de son évolution dans le temps,

  ledit premier capteur comprenant :
- un premier élément piézo-électrique réagissant en flexion et/ou en traction/compression aux déplacements d'un premier capteur-plaque en contact direct avec le corps dudit sujet et produisant une première différence de potentiel fonction desdits déplacements du premier capteur-plaque, et
- un second élément piézo-électrique de compensation réagissant en flexion et/ou en traction/compression aux déplacements d'un second capteur-plaque isolé du corps dudit sujet et produisant une seconde différence de potentiel fonction desdits déplacements du second capteur-plaque.

Ces capteurs piézo-électriques présentent l'avantage d'un faible encombrement et d'une bonne sensibilité. Ils sont de plus passifs, et sans nocivité physiologique.

Ainsi, l'invention permet de déterminer au moins une donnée représentative de la tension artérielle ou de la respiration, de façon simple et peu coûteuse. En effet, ces données ne sont pas obtenues directement, ainsi que cela se fait habituellement, mais à partir d'une analyse idoine du signal de pression sanguine instantanée, qu'il est aisé de relever et qui ne nécessite qu'une seule mesure sur le corps du patient.

Par ailleurs, le dispositif de l'invention permet de cette façon d'obtenir des informations en continu.

La qualité des informations est notamment assurée par une bonne élimination du bruit dû aux mouvements du patient. De façon avantageuse, lesdits moyens de réduction des perturbations comprennent :

- un différenciateur alimenté d'une part par ledit signal représentatif de la pression sanguine instantanée et d'autre part par ledit signal représentatif desdits mouvements, délivrant un premier signal corrigé, et

- des moyens de filtrage numérique adaptatif dudit premier signal corrigé, recherchant des corrélations entre ledit premier signal corrigé et ledit signal représentatif desdits mouvement et supprimant dans ledit premier signal corrigé les éléments correspondant auxdites corrélations, de façon à délivrer un second signal corrigé numérique.

Cette double correction permet d'effectuer une détermination fiable des informations de tension ou de respiration, le signal analysé étant ainsi très peu bruité.

- un différenciateur alimenté d'une part par ledit signal représentatif de la pression sanguine instantanée et d'autre part par ledit signal représentatif desdits mouvements, délivrant un premier signal corrigé, et
- des moyens de filtrage numérique adaptatif dudit premier signal corrigé, recherchant des corrélations entre ledit premier signal corrigé et ledit signal représentatif desdits mouvement et supprimant dans ledit premier signal corrigé les éléments correspondant auxdites corrélations, de façon a' délivrer un second signal corrigé numérique.

Cette double correction permet d'effectuer une détermination fiable des informations de tension ou de respiration, le signal analysé étant ainsi très peu bruité.

De façon avantageuse, lesdits premier et second éléments piézo-électriques sont sensiblement identiques et placés en superposition espacée. Ainsi, ils subissent sensiblement les mêmes mouvements.

Dans un mode de réalisation préférentiel de l'invention, ledit premier et/ou ledit second éléments piézo-électriques sont placés au-dessus et/ou au-dessous d'un évidement ménagé dans un circuit imprimé et fixés audit circuit imprimé par leurs extrémités, à l'aide d'une colle conductrice.

Dans le cas où le dispositif délivre au moins une information représentative de la tension artérielle relative, il comprend avantageusement des moyens de mesure de la pression d'application dudit premier capteur sur le corps dudit sujet, délivrant un signal représentatif de ladite pression d'application, lesdits moyens de traitement et d'analyse corrigent ladite information représentative de la tension artérielle relative en fonction dudit signal représentatif de la pression d'application.

En effet, cette pression d'application peut varier au cours du temps (mouvement du bracelet sur le poignet par exemple). Elle ne doit toutefois pas induire de modifications sur l'interprétation du signal mesuré. Les moyens de traitement effectue une correction, par exemple du type homothétique, en fonction de la pression d'application.

Cela est notamment utile lorsque le dispositif comprend des moyens de positionnement et/ou de fixation par serrage en contact avec le corps dudit sujet dudit boîtier, tel qu'un bracelet. Dans ce cas, lesdits moyens de mesure comprennent par exemple un capteur réagissant à la tension desdits moyens de positionnement et/ou de serrage.

Lesdits moyens de traitement et d'analyse peuvent notamment comprendre des moyens de calcul de la pression artérielle relative dudit sujet comprenant au moins un des moyens appartenant au groupe comprenant :

- des moyens de détection des extrema dudit signal représentatif de la pression sanguine instantanée sur une période de temps prédéterminée,
- des moyens de contrôle et de correction mettant en oeuvre un algorithme de vraisemblance,
- des moyens de correction de la pression artérielle relative en fonction de la pression exercée par ledit premier capteur sur le corps dudit sujet.

Avantageusement, le dispositif de l'invention comprend des moyens de détermination d'au moins une des informations représentatives de l'actimétrie dudit sujet appartenant au groupe comprenant :

- la vitesse moyenne,
- l'accélération moyenne,
  à partir dudit second signal représentatif des mouvements.

Ces données permettent notamment d'analyser plus finement les phases du sommeil, mais également de détecter par exemple le stress ou la maladie de Parkinson.

De façon avantageuse, le dispositif de l'invention comprend des moyens de détermination d'au moins une information représentative de la respiration dudit sujet, à partir d'une analyse des maxima et/ou des minima dudit signal représentatif de ladite pression sanguine instantanée.

Cette approche de la détermination de données respiratoires à partir de la pression sanguine est tout à fait nouvelle. Elle est basée notamment sur l'observation que la respiration module en amplitude et en fréquence la pression sanguine. Cela permet d'optimiser et d'étendre les applications de l'invention, par exemple à la détection de l'apnée.

Dans ce cas, le dispositif peut comprendre au moins un des moyens appartenant au groupe comprenant :

- des moyens de détection des maxima et/ou des minima dudit signal représentatif de ladite pression sanguine instantanée;
- des moyens de détermination et de suppression de la valeur moyenne desdits maxima, respectivement minima ;
- des moyens de calcul de la valeur absolue du signal formé par lesdits maxima, respectivement minima, représentatif de l'amplitude respiratoire ;

- des moyens de calcul de la période du signal formé par lesdits maxima, respectivement minima, représentative de la période respiratoire.

Avantageusement, le dispositif comprend encore des moyens de calcul du rythme cardiaque dudit sujet.

De tels moyens sont connus en eux-mêmes. Toutefois, l'invention propose un mode de réalisation nouveau particulièrement avantageux, dans lequel lesdits moyens de calcul du rythme cardiaque comprennent des moyens de détermination de la période du fondamental dudit signal représentatif de la pression sanguine instantanée.

Il peut par exemple s'agir de moyens de filtrage multicanal comprenant une pluralité de filtres possédant chacun une bande de fréquence distincte, et des moyens de détection du filtre correspondant à ladite période du fondamental.

Préférentiellement, le dispositif de l'invention comprend des moyens de mémorisation d'une série de valeurs de tension artérielle relative et/ou d'une série de valeurs de rythme cardiaque et/ou d'une série de valeurs représentatives de l'actimétrie et/ou d'une série de données représentatives de l'amplitude respiratoire et/ou d'une série de données représentatives de la période respiratoire.

Cela peut notamment permettre d'effectuer au moins un des traitements appartenant au groupe comprenant :

- calcul de moyennes et/ou de statistiques ;
- estimation et/ou prédiction de valeurs futures ;
- génération d'alarme en cas d'évolution alarmante.

Dans un mode de réalisation particulier, le dispositif comprend des moyens d'analyse d'au moins une desdites informations en fonction d'au moins une valeur de référence, de façon à fournir une information représentative d'un état dudit sujet, et par exemple d'une phase du sommeil.

Dans ce cas, le dispositif comprend avantageusement des moyens de programmation d'un instant approximatif et d'une phase de sommeil, permettant d'assurer un réveil lors de cette phase de sommeil.

Le dispositif peut comprendre de plus au moins un des moyens appartenant au groupe comprenant :

- moyens de visualisation ;
- horloge ;
- moyens de réveil ;
- moyens de programmation desdits moyens de réveil ;
- moyens de mémorisation ;
- moyens de communication avec une unité de traitement extérieur ;
- moyens de mesure de la température du corps et/ou du tonus musculaire dudit sujet.

Selon un premier mode préféré de réalisation de l'invention, destiné en particulier aux applications grand public, et plus généralement aux applications nécessitant une grande liberté de déplacement et/ou une untilisation sur une longue durée, l'ensemble des moyens constituant le dispositif de l'invention sont réunis dans un boîtier portatif autonome.

Selon un second mode préféré de réalisation, le dispositif comprend d'une part un boîtier portatif autonome, comprenant au moins lesdits capteurs, et d'autre part des moyens de traitement distants, comprenant au moins une partie desdits moyens de traitement et d'analyse, ledit boîtier comprenant de plus des moyens d'émission hertzienne et lesdits moyens de traitement distants comprenant de plus des moyens de réception, de façon à permettre la transmission des signaux relevés par lesdits capteurs.

Ce mode de réalisation correspond en particulier aux applications médicales (plusieurs boîtiers autonomes pouvant alors être contrôlés par les mêmes moyens de traitement), et plus généralement à toutes les applications nécessitant des capacités de stockage et/ou de traitement importantes.

Ce dispositif peut être utilisé pour de nombreuses applications, telles que :

- reconnaissance des phases du sommeil ;
- réveil du sujet dans une phase de sommeil prédéterminée et/ou dans un intervalle de temps déterminé ;
- détection de la somnolence ;
- quantification du sommeil ;
- aide à la détection des troubles du sommeil ;
- aide à la détection des apnées ;
- aide à la surveillance de maladies cardio-vasculaires ;
- aide à la programmation d'appareils en fonction des phases du sommeil ;
- aide à la analyse de la maladie de Parkinson ;
- aide à la prévention des ischémies cérébrales ;
- surveillance respiratoire ;
- surveillance de nourrisson ;
- analyse du sommeil polyphasique ;
- observation de la récupération après un effort ;
- suivi d'un niveau de vigilance ;
- aide à la détection du stress ;
- applications à l'élevage.

D'autres caractéristiques et avantages de l'invention apparaîtront à la lecture de la description suivante d'un mode de réalisation préférentiel de l'invention, donnée à titre illustratif et non limitatif, et des dessins annexés, dans lesquels :

- la figure 1 est un schéma synoptique présentant le principe général du dispositif de l'invention ;

- la figure 2 illustre un mode de réalisation avantageux de l'invention, dans lequel des capteurs piézo-électriques sont directement collés au circuit imprimé ;
- la figure 3 est le schéma électrique équivalent des capteurs piézo-électriques de la figure 2 ;
- la figure 4 présente les différents capteurs mis en oeuvre selon l'invention, dans le cas d'un dispositif se présentant sous la forme d'une montre-bracelet ;
- la figure 5 est un schéma synoptique détaillé d'un mode de réalisation avantageux de l'invention ;
- la figure 6 présente un exemple d'un signal de pression artérielle.
- la figure 7 illustre le principe de la reconnaissance des phases du sommeil (sur un exemple simplifié limité à la prise en compte des paramètres respiratoires) ;
- la figure 8 est un exemple de relevé obtenu à l'aide du dispositif de l'invention ;
- la figure 9 est un synoptique illustrant le fonctionnement du dispositif de l'invention.

L'invention concerne donc la détermination de signaux physiologiques, essentiellement à partir de l'analyse d'un signal représentatif des variations de la pression sanguine instantanée. Les paramètres que ce dispositif permet de déterminer sont notamment la fréquence cardiaque, la pression artérielle relative, l'amplitude respiratoire, la fréquence respiratoire, l'actimétrie.

Ces paramètres présentent en particulier les caractéristiques suivantes :

- fréquence cardiaque, exprimée en Hz, ou pouls, exprimé en nombre de battements/mn :

  - au repos pour un individu sain, il est de l'ordre de 70 ;
  - pour un sportif de haut niveau, la valeur varie en fonction de l'effort. Pour un cycliste, on a pu noter un pouls passant de 45 au repos à 180 en plein effort ;
  - en période de sommeil, la valeur du pouls varie. Elle est stable en sommeil lent profond et fluctue en sommeil profond. Les variations suivantes ont par exemple été rencontrées chez un individu sain : écart type inférieur à 2 pendant 20 mn dans la première phase de sommeil lent profond ; écart type supérieur à 5 pendant 30 mn de sommeil paradoxal.

  Suivant la méthode de calcul utilisée, il peut être plus simple de considérer la période de battement cardiaque (inverse de la fréquence cardiaque) exprimée en seconde et de calculer directement sa variation.
- pression artérielle relative : la pression artérielle est caractérisée par la valeur des pressions systolique (valeur haute) (SBP), diastolique (DBP) (valeur basse), et moyenne (MBP). Mais seules les variations de ces grandeurs sont représentatives du le stade de sommeil en cours. En conséquence, prendre en compte une valeur proportionnelle à SBP - DBP suffit.

  Une étude portant sur plusieurs personnes (12 sujets pendant 30 nuits), on a pu ainsi remarquer le long de la nuit une augmentation de pression moyenne de la SBP de 10 % et une variation de 10 % durant la première moitié de chaque phase de sommeil paradoxal. En faisant l'hypothèse que la structure des vaisseaux et le mode de fonctionnement du coeur ne sont pas modifiés le long de la nuit, une variation de SBP se traduit donc par une variation de SBP - DBP.
- amplitude respiratoire : on a constaté que le signal de pression sanguine est modulé en fréquence et en amplitude par l'effet de la respiration. La prise en compte de l'amplitude respiratoire se fait par la connaissance du taux de modulation an amplitude. En sommeil lent léger, le taux de modulation est faible (inférieur à 30 %). Il est important en sommeil lent profond (ronfleurs) et en paradoxal et peut dépasser 100 %. Il est stable en sommeil lent profond mais très variable en sommeil paradoxal.
- la fréquence respiratoire : quand on inspire le pouls augmente et quand on expire il diminue. En sommeil lent léger et en sommeil lent profond elle est très stable. On a pu remarquer pendant le sommeil paradoxal une variation de 2 cycles autour de la valeur moyenne de 14 cycles par 60 secondes, soit 14 % de variation.
- paramètres actimétriques : accélération et énergie. L'accélération existant dans les trois directions peut se réduire à l'accélération moyenne dans une direction privilégiée qui est environ celle perpendiculaire à la face interne du poignet pour rendre compte du mouvement de balancier.

  En intégrant l'accélération en fonction du temps, on obtient la vitesse de déplacement du poignet dans la direction précitée. Le carré de cette vitesse est fonction de l'énergie mise en jeu dans le mouvement du poignet.

  En cours d'éveil, l'accélération peut être faible alors que la vitesse est grande. En situation de stress, l'accélération est forte, et la vitesse faible. Les amplitudes observées sont propres à l'individu. En cours de sommeil lent profond, les paramètres actimétriques sont pratiquement nuls alors qu'en sommeil paradoxal ils sont importants (dans un rapport de 1 à 5).

Ainsi qu'on l'a déjà précisé, l'invention peut en particulier s'appliquer à la reconnaissance et/ou la quantification de phases du sommeil. C'est cette application qui est plus largement décrite par la suite, sans que cela soit limitatif de la portée de l'invention. Les mêmes démarches peuvent être mises en oeuvre pour toute autre application, et notamment celles listées par la suite.

On sait qu'une nuit de sommeil est divisible en 4 à 6 cycles, dont la durée varie de 60 à 120 minutes selon les individus, et selon l'état de fatigue ou de stress de ces individus. Chacun de ces cycles peut être décomposé en trois types de phases: le sommeil lent léger, le sommeil lent profond, le sommeil paradoxal et l'éveil.

Le sommeil lent léger se retrouve plusieurs fois par cycle. Il s'agit essentiellement d'une phase de transition entre l'éveil et une autre phase, ou entre deux autres phases.

Le sommeil lent profond apparaît surtout lors du premier cycle. Sa durée diminue fortement lors des cycles suivants. C'est le sommeil de récupération physique. Sa durée dépend des efforts de la journée. Ainsi, après une privation de sommeil, sa durée par rapport aux autres phases passe de 20 % à 40 %. La quantité de sommeil lent profond par nuit est sensiblement invariante suivant le type de dormeur (petit ou grand dormeur). Durant cette phase, la mémorisation est renforcée, et les rêves sont généralement constitués d'éléments rationnels brefs.

L'éveil à partir du sommeil lent profond est suivi d'une phase plus ou moins longue de confusion accompagnée de désorientation et de défaillance de la mémoire.

Le sommeil paradoxal constitue la principale période de rêves. Le tonus musculaire est alors totalement aboli, et des mouvements oculaires rapides sont perceptibles. Les rêves lors de cette période sont longs, animés et plus étranges que lors du sommeil profond. Ils appartiennent davantage au domaine de l'affectivité.

Un réveil pendant cette période de sommeil peut provoquer un stress. Par ailleurs, le corps est alors engourdi.

S'il y a réveil juste après le sommeil paradoxal, le sujet est présent et lucide. Il se souvient de ses rêves. Une augmentation de la durée de cette phase ne s'observe qu'après une privation de sommeil importante.

De nombreuses données physiologiques varient en fonction de ces phases du sommeil, et notamment :

- le rythme cardiaque ;
- la tension artérielle ;
- l'encéphalogramme ;
- la respiration ;
- le tonus musculaire ;
- les mouvements oculaires ;
- l'actimétrie (mouvements) ;
- ...

Ainsi qu'on l'a déjà signalé, le rythme cardiaque est une information très pertinente pour la reconnaissance des phases du sommeil. Toutefois, l'utilisation de cette seule information, telle que proposée dis la demande de brevet FR-A-2 679 453 déjà citée, et non encore publiée, ne permet pas d'obtenir un taux de bonne reconnaissance supérieur à 70%.

On sait qu'il existe une corrélation entre les variations de certaines grandeurs physiologiques. Les polysomnographes tiennent compte, d'ailleurs, de plusieurs de ces grandeurs pour effectuer la reconnaissance des phases.

Ces relations ont par exemple été étudiées dans les documents "Changes in respiration, heart rate, and systolic blood pressure in human sleep", Frederick SNYDER, J. Allan HOBSON, Donald F. MORRISON, and Frederick GOLDFRANK (National Institute of Mental Health, National Institutes of Health, Bethesda, Maryland. J. Appl. Physiol. 417-422 1964) et "Le sommeil humain, bases expérimentales physiologiques et physiopathologiques" Odile BENOIT et J.FORET (Edition Masson 1992).

Dans le premier document, on peut vérifier qu'il existe une corrélation certaine dans l'évolution du pouls et de la pression artérielle en fonction du stade du sommeil. Dans le second document, on montre notamment que des indéterminations entre l'éveil et certaines phases de sommeil peuvent être levées en tenant compte des pics tensionnels.

En particulier, on sait que la pression systolique moyenne baisse d'environ 5 à 15% en sommeil lent, que les les fluctuations de la pression systolique moyenne sont importantes, et qu'il apparaît une pointe tensionnelle à l'éveil.

Le problème des polysomnographes (outre leur coût et leur encombrement) est que la mesure des différentes grandeurs physiologiques nécessite la présence de capteurs sur le corps du sujet.

Ce problème est en particulier crucial pour la mesure de la tension artérielle. Cette mesure se fait classiquement à l'aide d'un garrot à serrage et déserrage progressifs nécessitant un capteur tenu par un brassard autour du bras ou d'un doigt et des moyens annexes de gestion.

Ce type de garrot présente plusieurs inconvénients : ils sont coûteux et encombrants, ils dérangent le dormeur dans son sommeil et surtout ils ne donnent pas d'information en continu. En effet, dans les polysomnographes, le garrot doit être activé à intervalle régulier (par exemple toutes les 10 minutes) pour délivrer une mesure. Aucune donnée n'est obtenue entre ces deux intervalles. Il n'est donc pas possible de connaître précisément l'instant de changement de phases, ou de détecter un réveil passager... Par ailleurs, sous l'effet du serrage du garrot, il est courant que le sujet se réveille (en particulier s'il souffre de troubles du sommeil). La mesure est alors faussée.

De même, la respiration varie en fonction des phases du sommeil, ainsi qu'on l'a déjà mentionné.

Selon l'invention, il est proposé de déterminer de la tension artérielle et/ou de données respiratoires en continu.

Cette approche repose en particulier, en ce qui concerne la tension artérielle, sur l'observation inventive que, dans de nombreuses applications,il n'est pas nécessaire de connaître les valeurs de tension artérielle de façon absolue, mais seulement leurs variations. En d'autres termes, l'homme du métier, et en particulier le médecin spécialiste du sommeil, a toujours considéré qu'il devait mesurer la tension du sujet. Les inventeurs ont vérifié cependant que la connaissance des différences de tension (mesure relative) était suffisante.

Cette approche nouvelle permet par ailleurs d'obtenir aisément la mesure de la tension en continu, en suivant les variations de pression sanguine.

Elle permet également, ainsi qu'on le verra par la suite, d'intégrer les moyens de mesure dans un boîtier de taille réduite, et en particulier dans une montre-bracelet.

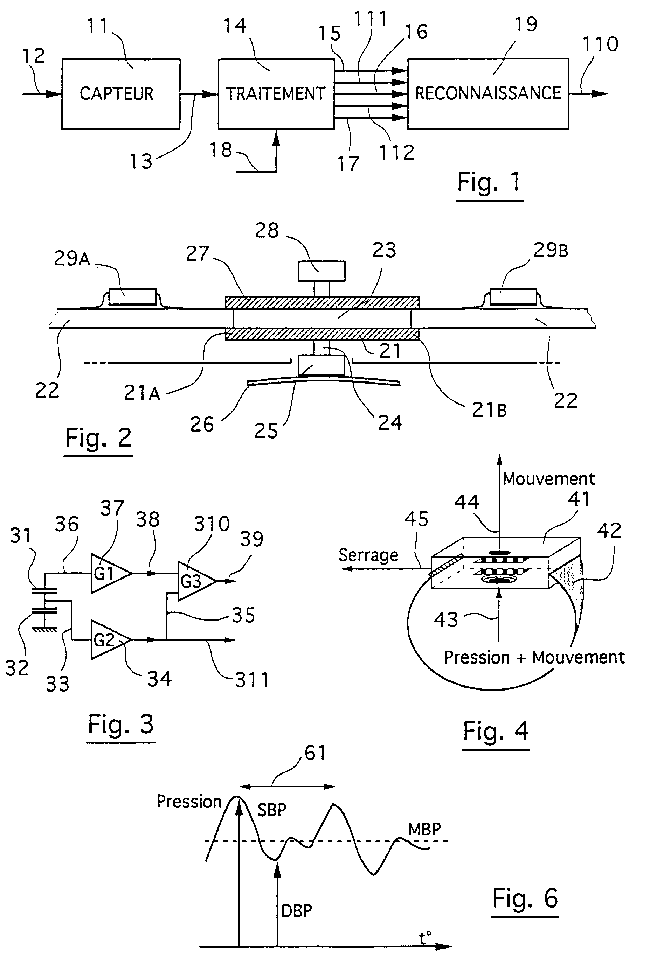

La figure 1 illustre le principe général du dispositif de l'invention.

Un capteur 11, destiné à être appliqué contre le corps du sujet, détecte les déplacements 12 de la partie du corps où il est appliqué. Le capteur 11 délivre un signal électrique 13, représentatif de ces déplacements.

Le capteur 11 détecte donc d'une part les mouvements du sujet, et d'autre part les déplacements de la peau correspondant aux variations de pression sanguine dans un conduit sanguin à proximité du capteur. Ainsi, le signal 13 comporte notamment une information représentative des variations de pression sanguine.

Ce signal 13 est ensuite traité dans des moyens de traitement 14, de façon à en extraire les informations suivantes :

- une information représentative de la tension artérielle 15 ;
- une information représentative du rythme cardiaque 16 ;
- une information représentative de l'actimétrie 17 ;
- une information représentative de l'amplitude respiratoire 111 ;
- une information représentative de la fréquence respiratoire 112.

Ainsi qu'on le verra par la suite, l'une de ces informations peut ne pas être considérée, dans un mode de réalisation dégradé.

En ce qui concerne l'information 15 de tension artérielle, un problème particulier est rencontré, dès lors que le dispositif de l'invention n'est pas parfaitement immobilisé contre le corps du sujet, par exemple à l'aide d'adhésifs... (c'est notamment le cas si le dispositif est fixé au bras par un bracelet) : la pression appliquée par le boîtier sur la peau du sujet peut varier, soit qu'il se déplace légèrement, soit que le serrage évolue (dans le cas d'un bracelet, celui-ci peut glisser le long du poignet vers un endroit où la circonférence du poignet est plus faible. La pression est alors moins forte).

Cette pression sur la peau a un lien direct avec l'amplitude du signal mesuré. On conçoit en effet que plus le boîtier exerce une pression sur la peau, plus les variations de pression sanguine présentent un niveau élevé. Ces variations de niveau ne doivent bien sûr pas être interprétée comme des variations de la pression sanguine.

Pour éviter ce problème, le dispositif de l'invention peut tenir compte d'une information 18 représentative de cette pression effectuée par le boîtier. Il peut par exemple s'agir d'une mesure de serrage du bracelet.

En d'autres termes, à pression de serrage du bracelet constante, la variation d'amplitude du signal est directement liée à la pression sanguine. Il suffit de réaliser une mesure d'amplitude du signal issu du capteur pour obtenir l'information sur la pression artérielle en s'assurant de la stabilité de la contrainte induite par le bracelet.

Plus généralement en mesurant la contrainte générée par le bracelet on peut corriger la mesure d'amplitude du signal qui deviendra une grandeur image de la pression sanguine. On utilise donc un capteur mesurant la contrainte induite par le bracelet.

Les informations 15 à 17 sont ensuite transmises à des moyens 19 d'analyse, et par exemple dans le mode de réalisation décrit, de reconnaissance des phases du sommeil. Ces moyens 19 de reconnaissance peuvent notamment comparer les informations 15 à 17 à différentes valeurs de seuil, déduites de la littérature. Avantageusement, des moyens de correction et d'ajustement sont utilisés, qui mettent par exemple en oeuvre un algorithme de vraisemblance. Ils peuvent également tenir compte de l'évolution dans le temps des informations 15 à 17. Un exemple de reconnaissance est décrit plus précisément par la suite.

Un dispositif selon l'invention peut donc comprendre :

- des moyens de mesure, regroupés dans un boîtier tenu au poignet ou en tout point du corps où les paramètres nécessaires peuvent être mesurés. Ce boîtier peut être tenu par un bracelet assurant un contact permanent avec le poignet. Les moyens de mesure comprennent notamment des capteurs (capteur de pression sanguine, capteur de mouvement, capteur de serrage du bracelet) et des moyens électroniques de conditionnement. La conversion A/N du signal peut se faire dans le boîtier ou au niveau des moyens de traitement selon l'architecture choisie ;
- des moyens de traitement, qui peuvent être externes (microordinateur), ou intégrés dans un ensemble de type ASIC placé dans le boîtier ou tout autre moyen permettant de dérouler un algorithme fonction de l'application ;
- une liaison entre les moyens précédents qui peut être soit un câble, soit une liaison optique ou hertzienne en modulation d'amplitude ou en modulation de fréquence. Cette liaison est indispensable lorsque les moyens de traitement ne sont pas inclus dans le boîtier.

Dans tous les cas, plusieurs boîtiers peuvent être couplés à un seul moyen de traitement et réciproquement.

La figure 2 représente (en coupe) un exemple de capteur d'activité cardiaque qui peut avantageusement être utilisé dans le dispositif décrit ci-dessus.

Il s'agit d'un capteur piézo-électrique captant le bruit, c'est-à-dire les changements de pression, provoqué par le passage du sang dans les veines, par exemple au niveau du poignet.

Un élément 21 en céramique piézo-électrique est collé par ses deux extrémités $21_A$, $21_B$ à un circuit imprimé 22, dans lequel il a préalablement été ménagé un jour 23 permettant à l'élément 21 de se déformer. Cet élément 21 a par exemple une taille de 12 x 4 x 0,7 mm.

La sensibilité de cette cellule piézo-électrique 21 est comprise entre 1 et 10 mm Hg, soit entre 1,3 et 13 kPa.

Une cale 24 est fixée, par collage, au centre de l'élément 21. Sur la cale 24 prend place une plaque 25 en aluminium, ou en tout autre matériau.

Cette plaque 25, qui peut avoir une surface de 7 x 7 mm, se trouve au contact de la peau 26 du sujet, lorsque le dispositif est en place. La large surface de contact permet d'obtenir un signal de mouvement de forte puissance.

Le mouvement de la peau est transmis à l'élément 21, par l'intermédiaire de la cale 24. Cela induit une flexion de l'élément, faisant apparaître une différence de potentiel entre les deux faces de l'élément 21. C'est cette différence de potentiel qui est fournie au module 14 de traitement du signal de la figure 1.

Avantageusement le capteur comporte un second groupe élément piézo-électrique 27 et plaque 28, isolé du corps. Ainsi la plaque libre 28 fournit un signal reflétant les mouvements du bras, mouvements qui induisent des signaux non utiles (parasites) dans le premier capteur. Une réduction de ces parasites peut être obtenue en combinant les signaux délivrés par les deux éléments 21 et 27 (soustraction du second au premier).

Le second groupe, ou "capteur de bruit parasite", peut également être positionné de façon différente, au niveau de la fixation du bracelet sur le boîtier. La céramique travaille alors en traction-compression et non plus en flexion, et capte ainsi mieux les perturbations produites par les variations de pression sur le premier capteur (toujours appliqué sur la peau) tout en étant isolé du signal utile (rythme cardiaque).

Dans le mode de réalisaiion de la figure 2, les deux bilames (ou éléments piézo-électriques) 21 et 27 sont identiques et compensées en accélération. Elles sont montées en opposition.

De façon à simplifier le montage et à limiter l'encombrement (et de réduire l'espace entre les deux cellules), les éléments 21 et 27 sont collés directement, par leurs extrémités, au circuit imprimé 22, à l'aide d'une colle conductrice. Ainsi, aucun moyen de calage ou de fixation n'est nécessaire, ni aucun moyen de câblage. D'autres techniques, plus classiques, peuvent également être utilisées.

Dans le même objectif de simplification du montage et de limitation de l'encombrement, les différents circuits électriques sont avantageusement des composants $29_A$, $29_B$ montés en surface (C.M.S).

Sous l'effet de l'accélération, on considère que les deux bilames ne se compensent qu'à 20%. On utilisera donc le signal de l'autre cellule pour corriger toute perturbation extérieure, sachant que les réponses impulsionnelles de chaque cellule sont identiques à un coefficient de proportionnalité près.

Le capteur peut donc être représenté, électriquement, par le schéma équivalent de la figure 3, associé à des amplificateurs.

Les cellules 21 et 27 correspondent à deux capacités en série 31 et 32. Le signal 33 prélevé sur la capacité 32 est amplifié par l'amplificateur 34, qui délivre un signal 35 représentatif des mouvements du sujet.

Le signal 36 prélevé sur la capacité 31 est également amplifié par un amplificateur 37 délivrant un signal 38 correspondant aux mouvements et aux variations de la pression artérielle.

Pour obtenir un signal 39 représentatif de la pression artérielle, le signal 35 (mouvements) est soustrait du signal 38 (mouvements + pression artérielle) par un différenciateur 310.

Si le dispositif prend également en compte l'actimétrie du sujet, le signal 35 est transmis (311) aux moyens de traitement. Dans ce cas, le signal 13 de la figure 1 correspond à la combinaison des signaux 39 et 311.

Les gains des amplificateurs sont, à titre indicatif, de l'ordre de :

- amplificateur 37 : G1 = 50
- amplificateur 34 : G2 = 200
- différenciateur 310 : G3= 1.

Les gains G1 et G2 sont différents car les sensibilités des deux capteurs sont différentes : du fait que la seconde cellule piézo-électrique est isolée, alors que la première subit la pression du poignet. Le gain G3 est choisi de façon à délivrer un signal présentant une bonne dynamique pour la conversion analogique/numérique.

Les cellules piézo-électriques peuvent par exemple être réalisées à partir de la céramique PXE 5 distribuée par RTC (marque déposée).

La figure 4 présente de façon schématique un dispositif selon l'invention, au format d'une montre-bracelet.

Ainsi qu'on l'a déjà mentionné, le dispositif global est avantageusement intégré dans un boîtier 41 maintenu au poignet à l'aide d'un bracelet 42.

Trois informations doivent donc être mesurées :

- l'ensemble pression + mouvement 43 ;
- le mouvement seul 44 ;
- la contrainte 45 effectuée par le bracelet sur le poignet (serrage) 45.

La mesure des données 43 et 44 a été décrite en liaison avec les figures 2 et 3. La cellule de mesure de la contrainte 45 doit être sensible à la composante continue. Plusieurs architectures sont possibles, et par exemple :

- un capteur passe-bas mesurant la composante continue et les fluctuations de la contrainte avec des sensibilités différentes ;
- un capteur passe-bas ne mesurant que la composante continue exercée par le poignet sur le capteur de pression artérielle ;
- un capteur passe-bas mesurant la contrainte du bracelet.

Sa dynamique doit être de l'ordre de 100 mm Hg soit 13 kPa. Cependant la détection de la contrainte 45 peut se faire à l'aide d'un capteur ou d'un capteur pression force moins sensible que le capteur de pression artérielle (par exemple une jauge piézo-résistive). Dans un mode de réalisation particulier, ce peut être des résistances déposées, telles que les résistances MOTOROLA (marque déposée) référencées MPX2040D. Il peut aussi s'agir d'un capteur déplacement sachant que celui-ci est fonction de la contrainte appliquée sur le poignet (l'allongement du bracelet correspond en effet à la position du bracelet, donc à la pression du boîtier sur la peau).

Dans ce dernier cas, un capteur de déplacement est fixé d'une part au boîtier 41, et d'autre part au bracelet 42. La tension subie par ce capteur est directement représentative du serrage.

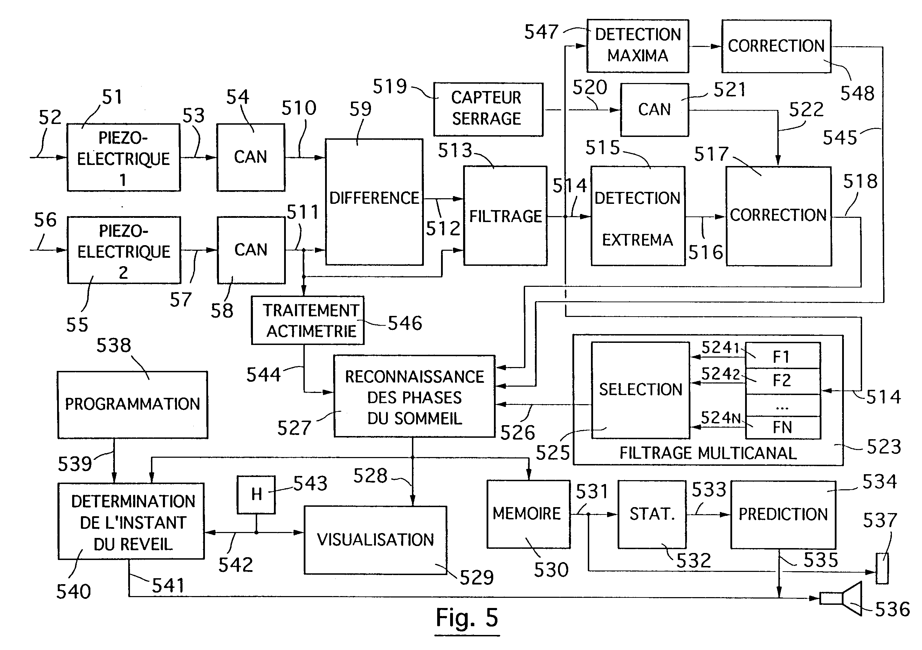

On présente maintenant un mode de réalisation particulier de l'invention, appliqué en particulier aux phases du sommeil, en liaison avec le schéma synoptique de la figure 5.

Un premier élément piézo-électrique 51 détecte donc des pressions 52 correspondant aux mouvements et à la pression artérielle. Il génère un signal électrique correspondant 53 qui est numérisé, à l'aide d'un convertisseur analogique/numérique 54.

De même, un second élément piézo-électrique 55 détecte les mouvements seuls 56. Le signal électrique correspondant 57 est également numérisé, par le convertisseur analogique/numérique 58.

Les deux CAN 54 et 58 échantillonnent les signaux par exemple à un rythme de 64 mesures/s. Ils peuvent avantageusement constituer deux voies d'un convertisseur se chargeant également de la conversion de la contrainte de serrage.

On effectue ensuite la différence 59 entre les signaux numérisés 510 et 511, pour obtenir le signal numérique 512 représentatif de la pression artérielle seule.

Dans la pratique, cette soustraction peut être double :

- on effectue tout d'abord une soustraction câblée, telle qu'illustrée en figure 4. En général, le signal résultant reste perturbé par les mouvements ;
- on réalise donc une seconde soustraction logicielle. Pour ce faire, un troisième convertisseur analogique/numérique numérise le signal "mouvements + pression artérielle" délivré par le premier élément piézo-électrique. Le traitement consiste alors à rechercher les corrélations entre les deux signaux numérisés, puis à supprimer du signal "pression artérielle" ce qui correspond aux mouvements.

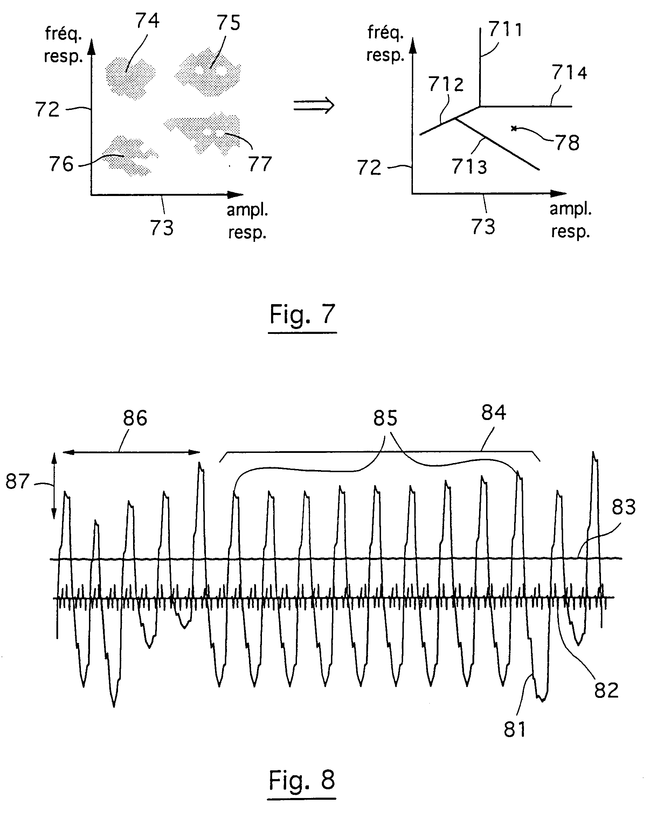

Ce signal 512 est transmis à un module 513 de linéarisation de filtrage passe-bas et/ou filtrage adaptatif et de mise à l'échelle. Dans le cas d'un filtrage adaptatif le module 513 est également alimenté par le signal 511, pour effectuer une recherche et une suppression des corrélations entre les deux signaux 511 et 512. La mise à l'échelle consiste à multiplier la valeur reçue par un coefficient de sensibilité représentatif du dispositif. Le signal correspondant 514 peut par exemple être tel que representé en figure 6.

Sur la figure 6, qui présente l'évolution de la pression artérielle en fonction du temps, on peut distinguer :

- la pression systolique (SBP) ;
- la pression diastolique (DBP) ;
- la pression artérielle moyenne (MBP).

Pour connaître ces différentes pressions on recherche les pics dans le signal 514, à l'aide d'un module 515 de détection des extrêma, sur une période paramétrable de quelques secondes. Connaissant ces extrêma 516, un module de correction 517 détermine une information 518 représentative de la tension artérielle relative du sujet (comprenant au moins une des données : SDP, DBP, MBP). La correction comprend deux niveaux :

- application d'un algorithme de vraisemblance (permettant notamment d'éliminer les données visiblement erronées) ;
- correction fonction de la contrainte exercée par le bracelet (serrage).

Pour ce deuxième niveau de correction, on met en oeuvre un capteur 519 de serrage (ou de pression), délivrant un signal 520 qui est numérisé (521) avant d'être transmis (522) au module 517 de correction.

Ainsi, sur une fenêtre glissante (de 8s par exemple), on calcule une grandeur qui est proportionnelle à la différence (SBP-DBP), à partir d'un filtrage passe-bande [0,5 Hz : 2 Hz signal de pression sanguine. Le résultat est redressé et filtré. Puis on en extrait la valeur moyenne. On détermine l'évolution du serrage du bracelet. Si le serrage du bracelet a changé, il faut établir une correction qui calcule l'amplitude du signal qui serait généré par la pression de serrage initial, et ceci par homothétie. Puis on détermine la variation par comparaison à la valeur moyenne précédente, qui est le résultat cherché.

On détermine ensuite le rythme cardiaque (ou pouls), à partir de l'information 514 de tension artérielle. Cette opération consiste à mesurer la période du fondamental (61, figure 6) contenu dans le signal de pression artérielle.

Plusieurs méthodes sont utilisables et nécessitent des moyens de calcul et de mémoires différents. Avec un traitement fréquentiel, on pourra utiliser une analyse spectrale ou une méthode dérivée. Avec un traitement dans le domaine temporel on utilisera un calcul d'autocorrélation à durée variable par exemple.

En remarquant que la bande d'analyse est étroite et qu'il y a uniquement recherche du fondamental, une approche de calcul par filtrage multicanal 523 est souhaitable. En effet la bande d'analyse est de 30 battements/s à 240 battements/s soit 0,5Hz à 4Hz. Cette méthode a la particularité d'être peu gourmande en capacité mémoire et en nombre d'opérations à exécuter compte tenu des fréquences de chaque canal $524_1$ à $524_N$ qui peuvent être 0,5 0,66 0,8 1 1,33 2 4 Hz, correspondant à des rythmes cardiaques de 30, 40, 48, 60, 80, 120 et 240 battements/mn.

A chaque canal correspond un débit sous-multiple de la cadence maximale. Ainsi on n'utilise qu'un seul gabarit de filtre. A la sortie de chaque canal $524_1$ à $524_N$ on calcule (525) l'énergie du signal et la période du signal filtré, par comptage. On obtient donc un spectre de raies d'énergie et une fonction de périodes calculées. Une stratégie de sélection 525 détermine le canal le plus fiable, et délivre l'information correspondante 526 au module 527 de reconnaissance des phases du sommeil.

Plus généralement, la méthode consiste à, sur une fenêtre glissante de durée 8 s par exemple, ou de même durée que la période respiratoire, appliquer un opérateur mathématique qui calcule la période ou la fréquence du signal de pression sanguine, puis la variance à partir de quoi on estimera l'écart-type ou variabilité. Les opérateurs mathématiques peuvent être :

- une fonction d'autocorrélation,
- un filtrage multicanal,
- un calcul d'autocorrélation partielle avec suivi des pics mini et maxi,
- une estimation spectrale.

On calcule ensuite les paramètres respiratoires 545. On cherche (547) les maxima du signal de pression sanguine (amplitude et coordonnées dans le temps). Puis, on supprime (548) la valeur moyenne et, en détectant les passages à zéro du signal, on calcule la période respiratoire 545 et son inverse, la fréquence. Sur la durée de cette période, on calcule la valeur moyenne du signal redressé qui est une bonne image de l'amplitude respiratoire 545.

On peut opérer de même avec les minima, et moyenner les résultats obtenus entre les minima et les maxima.

Pour obtenir de façon satisfaisante le calcul des paramètres actimétriques, on réalise 546 un sous-échantillonnage (1/8) sur le signal de mouvement puis un redressement et un filtrage passe-bas. La valeur moyenne calculée est proportionnelle à l'amplitude de l'accélération moyenne pendant une fenêtre donnée, dans la direction perpendiculaire à l'intérieur du poignet.

L'énergie du mouvement est calculée après intégration du signal 511 sous-échantillonné. Ensuite, on redresse le signal et on lui applique un filtre passe-bas et on calcule la moyenne qui est proportionnelle à la vitesse moyenne, dans la direction précédente et sur la même fenêtre. L'énergie, proportionnelle au carré de la vitesse, est alors calculée si nécessaire.

Le module 527 reçoit donc quatre informations :

- pression artérielle 518 ;
- rythme cardiaque 526 ;
- actimétrie 544 ;
- paramètres respiratoires (fréquence et amplitude) 545.

Dans des modes de réalisation simplifiés, il est bien sûr possible de ne pas tenir compte de l'ensemble de ces informations.

La validité de ces informations a été vérifiée en milieu médical, notamment au CHR Kergormar de Lannion et au CHR de Brest, par comparaison avec des signaux mesurés à l'aide de capteurs médicaux classiques (sysmographes, respirographes, ...).

Le module 527 calcule les variations de différentes informations qui permettent de déterminer la phase du sommeil.

Chacune de ces informations est par exemple comparée à un ou plusieurs seuils caractéristiques des différentes phases de sommeil. Les résultats de ces comparaisons sont ensuite corrélés entre eux pour déterminer la phase de sommeil 528 en cours. Avantageusement, un algorithme de vraisemblance est mis en oeuvre pour contrôler la fiabilité de la reconnaissance.

Cette information 528 de phase de sommeil peut être visualisée, par exemple à l'aide d'un écran 529 à cristaux liquides. Elle est également mémorisée, dans des moyens 530 de stockage. Ceux-ci peuvent également stocker les différentes informations 511, 518 et 527, 544, 545.

Plus généralement, les sorties peuvent être :

- un affichage de type montre,
- un réveil sonore ou lumineux,
- une trace papier,
- une mémorisation,
- une commande d'appareils.
- etc ...

Les données stockées 531 permettent d'effectuer des stastistiques 532, dont les résultats 533 peuvent être visualisés, mais surtout utilisés dans un module 534 de prédiction. Il est ainsi possible de générer un signal d'alarme 535, produisant un signal sonore 536, lorsque l'évolution de la pression artérielle et/ou du rythme cardiaque présente des caractéristiques dangereuses pour le sujet. Il est à noter que ce module de prédiction est très important, car ce ne sont pas les valeurs absolues des signaux mais leurs évolutions dans le temps qui sont significatives.

Pour des analyses médicales plus poussées, le dispositif peut comprendre des moyens de connexion 537, qui permettent de transmettre le contenu de la mémoire 530 vers une unité de traitement externe.

Eventuellement la liaison peut être hertzienne.

Par exemple, la liaison HF peut mettre en oeuvre une modulation d'amplitude (mode ASK). L'émetteur (dans le boîtier) comprend par exemple un oscillateur local à onde de surface vibrant à 224,5 MHz. A cette fréquence, l'antenne d'émission est de dimensions réduites (quelques cm de côté) et peut être réalisée sur le circuit imprimé.

Après la transmission d'une mesure, l'émetteur est mis en veille, ce qui réduit la consommation de courant. La puissance de l'émetteur peut être inférieure à 5 mW et sa portée de quelques mètres.

Pour le récepteur (dans les moyens externes), on pourra choisir un récepteur de type "super-réaction" avec un oscillateur local à 213,8 MHz et une amplification intermédiaire à 10,7 MHz suivi d'un décodage.

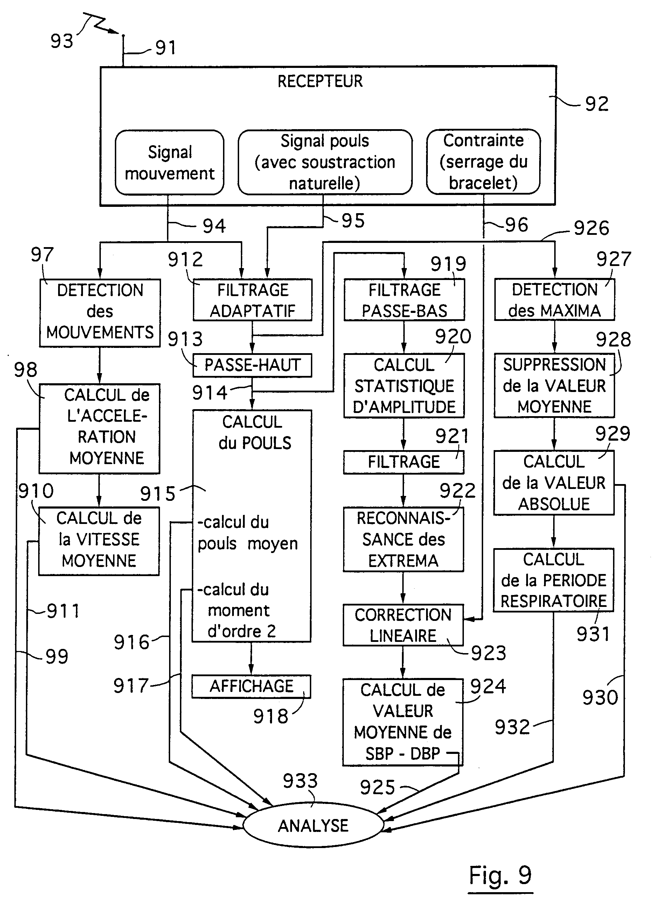

La figure 9 présente, sous une autre forme, les traitements effectués dans un dispositif selon l'invention.

Dans cette exemple, les moyens de traitement sont désolidarisés du boîtier autonome portant les capteurs (le même traitement peut bien sûr être effectué en interne directement dans le boîtier). Les mesures effectuées sont reçues par voie hertzienne à l'aide d'une antenne 91. Un récepteur/décodeur 92 restitue, à partir du signal reçu 93, un signal représentatif du mouvement 94, un signal représentatif du pouls 95 (correspondant à la soustraction des signaux des deux capteurs) et un signal représentatif de la contrainte (serrage du bracelet) 96.

Quatre traitements distincts sont alors effectués :

- actimétrie : à partir du signal de mouvement 94, on détecte les mouvements (97), dont on déduit (98) l'accélération moyenne 99, par intégration de la valeur absolue du signal de mouvement, puis (910) la vitesse moyenne 911 de la valeur absolue du signal, proportionnel à l'énergie ;
- pouls : à partir du signal de pouls 95 et du signal de mouvement 94, on effectue un filtrage adaptatif 912 de soustraction du bruit résiduel dû aux mouvements, puis un filtrage passe-haut 913 pour supprimer la basse fréquence résiduelle. Le signal filtré 914 permet de calculer (915) le pouls moyen 916 et le moment d'ordre 2 917 représentatif du pouls moyen. Eventuellement, le pouls moyen peut être affiché (918) par exemple toutes les 10 ou 20 secondes.
- tension artérielle relative : le signal filtré 914 utilisé pour le pouls est également utilisé pour la tension. Il subit tout d'abord un filtrage 919 passe-bas complémentaire d'affinage de la bande à analyser, puis un calcul statistique d'amplitude 920 (histogramme), un filtrage 921 et une détection 922 des extrema. On pratique ensuite une correction linéaire 923 du signal obtenu en fonction de la contrainte, c'est-à-dire de l'information de serrage 96. Enfin, on effectue (924) le calcul de la valeur moyenne de (SBP-DBP) 925.
- respiration : le signal 926 représentatif du pouls obtenu après la double soustraction du bruit est soumis à une détection des extrema 927, puis à une suppression 928 de la valeur moyenne. Ensuite, on calcule (929) la valeur moyenne du signal, qui fournit une information 930 représentative de l'amplitude respiratoire, et (931) la période du signal, qui correspond à la période respiratoire 932.

L'ensemble des informations calculés 911, 916, 917, 925, 930, 931 (ou une sélection de ceux-ci, en fonction de l'application) est transmis à un module d'analyse 933, qui effectue l'analyse correspondant à l'application.

La figure 7 illustre un exemple de fonctionnement de ce module 923 dans le cas de la reconnaissance des phases de sommeil.

On se limite, pour des raisons de simplicité de représentation (espace à deux dimensions), au cas des données respiratoires afin de détailler le processus de classification. La généralisation à un espace à n dimensions est directe. L'extraction de données effectuée sur la fenêtre temporelle étudiée donne deux variables relatives à la respiration comme il est indiqué ci-dessus. Le processus de reconnaissance fonctionne en deux parties :

- la première partie, appelée "apprentissage", est effectuée avant d'essayer de reconnaître les phases d'une nuit de sommeil d'un patient. Dans cette partie, on regarde la répartition des différentes mesures effectuées sur ces deux paramètres respiratoires, dans un espace de représentation qui a pour axe les deux variables fréquence respiratoire 72 et amplitude respiratoire 73. On sait par ailleurs l'appartenance exacte de toutes ces mesures aux différentes phases du sommeil (éveil 74, sommeil paradoxal 75, sommeil lent léger 76, sommeil lent profond 77).

On peut donc tracer dans cet espace des limites $71_1$ à $71_4$ qui déterminent les différentes classes possibles pour les vecteurs de mesure formés par ces deux variables, ainsi que cela est illustré en figure 7. Si l'on suppose que les classes sont linéairement séparables, les limites sont alors des segments de droite $71_1$ à $71_4$. Ces segments de droites sont obtenus par des algorithmes de type gradient ou encore par l'algorithme du "perceptron", bien connus en reconnaissance de formes. On peut aussi les tracer directement à vue d'oeil. D'autres types de limites sont encore utilisables et celles-ci ne se limitent pas à des figures géométriques, mais peuvent aussi être exprimées statistiquement.

- la seconde partie appelée "reconnaissance" ou "classification" consiste simplement à localiser dans cet espace de représentation le point représentatif de la mesure que l'on cherche à classer, par rapport aux différentes limites, 78. La déclaration d'identité de la phase reconnue est alors immédiate.

On obtient donc après cette première partie du traitement, trois déclarations d'identité concernant le phase de sommeil que l'on veut reconnaître, respectivement la déclaration De sur les variables cardiaques, Dr sur les variables de la respiration, et Dm sur le mouvement. On a de plus la déclaration de phase précédente D(t-1). Pour combiner (fusionner) ces différentes déclarations, on peut par exemple utiliser une méthode de scrutin majoritaire entre les différentes déclarations : D(t) = MAJ [Dc, Dr, Dm, D(t-1)] et vérifier par relations logiques si le passage de D(t-1) à D(t) n'est pas invraisemblable. D'autres méthodes (heuristiques, statistiques. ...) peuvent être effectuées pour la fusion.

Finalement, après traitement de toutes les mesures effectuées pendant la nuit, on obtient l'instant d'occurrence et la durée de chaque phase de sommeil. Il est à noter que les différentes déclarations (sur chaque groupe et par conséquent la déclaration de fusion) peuvent être données avec un facteur de confiance associé. Ce facteur indique la confiance que l'on accorde au résultat et peut être calculé à partir de l'évaluation des performances des classifieurs réalisés.

Parmi les nombreuses applications médicales de l'invention, on peut notamment citer :

- reconnaissance des stades de sommeil : un taux de reconnaissance de l'ordre de 85% peut être atteint, qui ouvre le champs à des nouvelles applications.
- application à la somnolence : cette application est très étendue et nécessite une recherche conséquente ainsi que la réalisation de mesures comparatives. L'état de somnolence est un état transitionnel entre l'éveil et le sommeil lent léger - stade 1. L'activité cardiaque est modifiée et le traitement de nouvelles informations réalisé par un capteur facilement portable serait très apprécié.
- quantification du sommeil. A partir d'une bonne reconnaissance on peut sommer la durée des différents stades, apprécier les différents sommeils en nature et en durée et déduire d'éventuels troubles. L'analyse du sommeil le long de la nuit peut faciliter un prédiagnostique dans certaines maladies (apnée du sommeil) par exemple.
- Suivi à court terme de certaines maladies cardio-vasculaires. Le généraliste ayant un patient en consultation lui mesure la tension et lui propose le port du dispositif pendant quelques jours. Partant de sa mesure et des enregistrements faits par le dispositif il peut déduire l'évolution de la pression absolue du patient.

Les maladies cardiaques sont la principale cause d'incapacités permanentes chez les adultes agés de moins de 65 ans (elles sont responsables de 50% des jours d'hospitalisation aux USA), d'après "Les maladies cardiaques, l'hypertension & l'alimentation" de Alison Hull (éditions du Trécarré). La prévention de celles-ci par un appareil portable et convivial représente donc une avancée dans le dépistage de ces maladies.

Le dispositif selon l'invention peut également permettre de programmer une heure biologique de réveil. En relation avec la figure 5, des moyens de programmation 538 permettent de définir un intervalle de temps et une phase de sommeil 539 pendant laquelle on souhaite être réveillé.

Par exemple, si le réveil est souhaité en corrélation avec une phase de sommeil, il est possible de programmer ce réveil en précisant un créneau horaire et la phase en cours souhaité. Par exemple, si on veut être réveillé en sommeil lent léger à 7 H, on précisera SLL - 6H40 - 7H15. La programmation du réveil se fait par boutons poussoirs comme sur une montre classique.

Un module 540 de détermination de l'instant du réveil peut alors générer une alarme 541, en fonction de cette programmation 539, de la phase du sommeil reconnue 528 et de l'heure 542 fournie par une horloge 543 (qui offre également avantageusement toutes les fonctions d'une montre).

Cette seconde application, qui peut être cumulée ou distincte des précédentes, peut par exemple intéresser :

- les médecins hypnologues (traitant des troubles du sommeil) pour les problèmes de leurs patients, qui ne peuvent tenir un compte de leurs heures de sommeil. Une ou plusieurs nuits à l'hôpital sont jusqu'ici nécessaire pour recueillir des données scientifiques. Cela coûte évidemment très cher pour une fiabilité biaisé, car on sort le patient de son environnement, ce qui perturbe fortement son sommeil ;
- les médecins cardiologues, pour suivre l'effort cardiaque de leurs patients tout au long de la journée ;
- les particuliers ayant des surcharges de travail pendant quelques jours, et voudraient ne pas perdre de temps à rêver (étudiants, professions libérales, militaires ou navigateurs...) ;
- les malades cardiaques, qui doivent limiter leurs efforts. L'invention peut signaler un dépassement de seuil ou de fatigue accumulée ;
- les personnes souhaitant fractionner leurs temps de sommeil et faire une sieste, et voulant limiter la sieste au cycle le plus important et avoir une sieste courte et un réveil rapide ;
- les personnes ayant du mal à se lever !
- les personnes portant intérêt à leurs rêves ;
- les personnes qui veulent pouvoir gérer au mieux leurs efforts, leur récupération et leur alimentation ;
- etc...

D'autres applications de l'invention peuvent être encore :

- l'apprentissage. Certaines périodes de sommeil (sommeil paradoxal) sont propices à la réception d'informations scolaires ou autres. Le déclenchement d'un moyen sonore en cours de sommeil paradoxal est possible.
- la quantification du sommeil. La quantité du sommeil (durée et structure) est un élément déterminant pour observer sa forme. C'est pourquoi il est intéressant de connaître la structure de son sommeil. Savoir que le sommeil n'a pas été conséquent peut induire à chercher un sommeil plus long et éviter une fatigue. Ceci est possible car le dispositif peut garder en mémoire le tracé de la nuit et peut l'éditer.
- aide au prédiagnostic des apnées (à domicile ou en milieu hospitalier). La courbe de la figure 8 montre l'évolution des 3 signaux (81 - pression sanguine, 82 - mouvement, 83 - serrage du bracelet) au cours d'une période de nuit. On observe que durant 8 impulsions cardiaques, le signal de pression sanguine est plat 84 (absence de modulation), ce qui montre l'arrêt de la respiration. En même temps, l'amplitude 85 de ce signal augmente de 25 % alors que le serrage de bracelet est stable. Ceci signifie que l'arrêt respiratoire s'accompagne d'une augmentation de la pression artérielle. On distuingue également aisément sur ce schéma la demie-période respiratoire 86 et l'amplitude respiratoire 87 (dans la pratique, ces données sont bien sûr déterminées sur une durée plus longue).

Ceci a déjà été observé lors d'apnée mais jamais par des mesures en continu (uniquement par garrottage), ce qui limite l'interprétation des mesures. Pour le moment, l'apnée est détectée à l'aide d'appareillage lourd et uniquement en milieu hospitalier.

Il est à noter que, pour déterminer l'arrêt respiratoire, la connaissance du serrage du bracelet n'est pas utile. Par contre, pour observer l'augmentation de pression artérielle, elle est nécessaire.

- aide au diagnostic de la maladie de PARKINSON ; cette maladie peut être diagnostiquée au long d'une nuit par actimétrie simple, mais l'invention représente une amélioration puisqu'il permet de préciser la structure typique du sommeil du malade ;
- prévention des ischémies cérébrales. Les ischémies cérébrales ont lieu chez certains sujets (personnes souffrant d'hypertension ou âgées, etc...) pendant les phases de sommeil paradoxal et surtout en fin de nuit. S'il y a intervention dans les 6H, le risque de voir les lésions évoluer de façon irréversible est limité.
- surveillance respiratoire. Elle peut être utile dans certaines conditions (en milieu hospitalier ou a domicile). Pour cela, une seule cellule piézoélectrique suffit, s'il n'y a pas de corrélation à rechercher avec le sommeil.
- surveillance de nourrisson. Pour les familles qui ont connaissance des risques encourus par leur nourrisson, il est possible de réaliser un suivi de nourrisson avec la possibilité de déclencher une alarme en fonction de l'évolution de l'un des paramètres biologiques.
- détection de l'état de somnolence. Pour certaines personnes, il est intéressant de réaliser l'enregistrement diurne des paramètres biologiques sachant qu'elles souffrent de somnolence, cet état pouvant être l'indicateur d'une situation dépressive.
- suivi de l'effet de certains médicaments. Plus généralement, avant d'ordonner un traitement, le généraliste peut vouloir prendre connaissance de la structure du sommeil d'un patient souffrant de mauvaise nuit. Ensuite, il ordonnera un traitement et il pourra suivre l'effet des médicaments et adapter au mieux ce traitement (économie de médicaments). Pour cela, il ordonnera le port d'un dispositif selon l'invention à domicile pendant quelques nuits, pour réaliser un enregistrement ambulatoire.
- applications sportives :

- sommeil polyphasique
- observation de la récupération après un effort prolongé

- applications professionnelles humaines :
  Suivi du niveau de vigilance :

  - pour le travail posté fatiguant et dangereux
  - pour la conduite automobile

- aide au détection du stress et traitement à partir d'un environnement crée et variable (musique, sons, couleurs,...) en fonction du stress mesuré.
- applications professionnelles à l'élevage. Les abatteurs de bovins aiment être assurés que les bêtes à tuer ne sont pas stressées. Car si tel est le cas, la viande est dure et le pH est mauvais et tel que la viande puisse être refusée au contrôle vétérinaire. De même, les abatteurs de porcs aiment savoir que les porcs abattus ont eu une vie "normale" (pas de maladie donc pas de traitement). En suivant le sommeil de la bête et sa température, ils peuvent prétendre traiter une viande d'une qualité connue. En outre, les comportements diurne et nocturne de l'animal sont fonction de l'alimentation, d'où l'avantage qui peut être retiré du suivi comportemental de l'animal.

Par ailleurs, de nombreuses adaptations de l'invention sont envisageables, tant en ce qui concerne les types du ou des capteurs utilisés, les supports de l'invention (bracelets, bagues, colliers, systèmes adhésifs, etc...) ou les types de traitements et/ou de calculs effectués à partir des signaux issus des capteurs.

**Revendications**

1. Dispositif de détermination d'informations physiologiques comprenant :

   - un premier capteur (11 ; 21; 51) adapté pour être placé en contact avec la peau d'un sujet et réagissant aux variations (12 ; 56) de la pression sanguine instantanée dudit sujet, délivrant un signal représentatif (13 ; 53 ; 95) de ladite pression sanguine instantanée, et
   - un second capteur (28 ; 55) isolé de la peau dudit sujet et réagissant aux mouvements dudit sujet, délivrant un signal (57 ; 94) représentatif desdits mouvements,
     caractérisé en ce qu'il comprend :
   - des moyens (14 : 59, 513 ; 912) de réduction des perturbations présentes dans ledit signal représentatif (13 : 53 ; 95) de la pression sanguine instantanée dues auxdits mouvements, en fonction dudit signal représentatif (57 ; 94) desdits mouvements, lesdits moyens de réduction délivrant un signal corrigé, et

- des moyens de traitement et d'analyse (14 : 515, 517, 546, 547, 548, 523 ; 98, 910, 913, 915, 919, 920, 921, 922, 923, 924, 927, 928, 929, 931) dudit signal corrigé, délivrant au moins une des informations appartenant au groupe comprenant :

  - une information (15 ; 518 ; 925) représentative de la tension artérielle relative dudit sujet et/ou de son évolution dans le temps ;
  - une information (112 ; 545 ; 932) représentative de la fréquence respiratoire dudit sujet et/ou de son évolution dans le temps ;
  - une information (111 ; 545 ; 930) représentative de l'amplitude respiratoire dudit sujet et/ou de son évolution dans le temps,

  et en ce que ledit premier capteur (11) comprend :
  - un premier élément piézo-électrique (21) réagissant en flexion et/ou en traction/compression aux déplacements d'un premier capteur-plaque (24) en contact direct avec le corps dudit sujet et produisant une première différence de potentiel fonction desdits déplacements du premier capteur-plaque (24), et
  - un second élément piézo-électrique (27) de compensation réagissant en flexion et/ou en traction/compression aux déplacements d'un second capteur-plaque (28) isolé du corps dudit sujet et produisant une seconde différence de potentiel fonction desdits déplacements du second capteur-plaque (28).

2. Dispositif selon la revendication 1, caractérisé en ce que lesdits moyens de réduction des perturbations comprennent :

   - un différenciateur (310 ; 59) alimenté d'une part par ledit signal représentatif (13 ; 311 ; 510) de la pression sanguine instantanée et d'autre part par ledit signal représentatif (38 ; 511) desdits mouvements, délivrant un premier signal corrigé (39 ; 512 ; 95), et
   - des moyens (513 ; 912) de filtrage numérique adaptatif dudit premier signal corrigé, recherchant des corrélations entre ledit premier signal corrigé et ledit signal représentatif desdits mouvement et supprimant dans ledit premier signal corrigé les éléments correspondant auxdites corrélations, de façon à délivrer un second signal corrigé numérique (514 ; 926).

3. Dispositif selon l'une quelconque des revendications 1 et 2, caractérisé en ce que lesdits premier et second éléments piézo-electriques (21, 27) sont sensiblement identiques et placés en superposition espacée.

4. Dispositif selon l'une quelconque des revendications 1 à 3, caractérisé en ce que ledit premier et/ou ledit second éléments piézo-électriques (21, 27) sont placés au-dessus et/ou au-dessous d'un évidement (23) ménagé dans un circuit imprimé (22) et fixés audit circuit imprimé (22) par leurs extrémités (21$_A$, 21$_B$).

5. Dispositif selon la revendication 4, caractérisé en ce que ledit premier et/ou ledit second éléments piézo-electriques (21, 27) sont collés audit circuit imprimé (22) par leurs extrémités (21$_A$, 21$_B$), à l'aide d'une colle conductrice.

6. Dispositif selon l'une quelconque des revendications 1 à 5 délivrant au moins une information (15 ; 518 ; 925) représentative de la tension artérielle relative, caractérisé en ce qu'il comprend des moyens (519) de mesure de la pression d'application (45) dudit premier capteur (21 ; 51) sur le corps dudit sujet, délivrant un signal (18 ; 520 ; 96) représentatif de ladite pression d'application,
et en ce que lesdits moyens de traitement et d'analyse (14 ; 517 ; 923) corrigent ladite information représentative de la tension artérielle relative (15 ; 516) en fonction dudit signal représentatif (18 ; 520 ; 96) de la pression d'application.

7. Dispositif selon la revendication 6, caractérisé en ce qu'il comprend des moyens (42) de positionnement et/ou de fixation par serrage en contact avec le corps dudit sujet dudit boîtier (41), et en ce que lesdits moyens de mesure (519) comprennent un capteur réagissant à la tension desdits moyens (42) de positionnement et/ou de serrage.

8. Dispositif selon l'une quelconque des revendications 1 à 7, caractérisé en ce que lesdits moyens de traitement et d'analyse comprennent des moyens (515, 517 ; 922, 923) de calcul de la pression artérielle relative (518) dudit sujet comprenant au moins un des moyens appartenant au groupe comprenant :

- des moyens (515 ; 922) de détection des extrema (516) dudit signal (514) représentatif de la pression sanguine instantanée sur une période de temps prédéterminée,
- des moyens (517) de contrôle et de correction mettant en oeuvre un algorithme de vraisemblance,
- des moyens (517 ; 923) de correction de la pression artérielle relative en fonction de la pression exercée (520 ; 96) par ledit premier capteur sur le corps dudit sujet.

9. Dispositif selon l'une quelconque des revendications 1 à 8, caractérisé en ce qu'il comprend des moyens de détermination d'au moins une des informations (17 ; 99, 911) représentative de l'actimétrie dudit sujet appartenant au groupe comprenant :

- la vitesse moyenne (911),
- l'accélération moyenne (99),
  à partir dudit second signal représentatif des mouvements.

10. Dispositif selon l'une quelconque des revendications 1 à 9, caractérisé en ce qu'il comprend des moyens de détermination d'au moins une information (111, 112 ; 545 ; 930, 932) représentative de la respiration dudit sujet, à partir d'une analyse (547, 548 ; 927, 928, 929, 931) des maxima et/ou des minima dudit signal représentatif de ladite pression sanguine instantanée.

11. Dispositif selon la revendication 10, caractérisé en ce qu'il comprend au moins un des moyens appartenant au groupe comprenant :

- des moyens (547 ; 927) de détection des maxima et/ou des minima dudit signal représentatif (13) de ladite pression sanguine instantanée ;
- des moyens (548 ; 928) de détermination et de suppression de la valeur moyenne desdits maxima, respectivement minima ;
- des moyens (548 ; 929) de calcul de la valeur absolue du signal formé par lesdits maxima, respectivement minima, représentatif de l'amplitude respiratoire ;
- des moyens (548 ; 931) de calcul de la période du signal formé par lesdits maxima, respectivement minima, représentative de la période respiratoire.

12. Dispositif selon l'une quelconque des revendications 1 à 11, caractérisé en ce qu'il comprend des moyens (523 ; 915) de calcul du rythme cardiaque (526 ; 916) dudit sujet.

13. Dispositif selon la revendication 12, caractérisé en ce que lesdits moyens de calcul du rythme cardiaque comprennent des moyens (523) de détermination de la période du fondamental dudit signal (514) représentatif de la pression sanguine instantanée.

14. Dispositif selon la revendication 13, caractérisé en ce que lesdits moyens de détermination comprennent des moyens (523) de filtrage multicanal comprenant une pluralité de filtres (524$_1$ à 524$_N$) possédant chacun une bande de fréquence distincte, et des moyens (525) de détection du filtre correspondant à ladite période du fondamental.

**15.** Dispositif selon l'une quelconque des revendications 1 à 14, caractérisé en ce qu'il comprend des moyens (530) de mémorisation d'une série de valeurs de tension artérielle relative et/ou d'une série de valeurs de rythme cardiaque et/ou d'une série de valeurs représentatives de l'actimétrie et/ou d'une série de données représentatives de l'amplitude respiratoire et/ou d'une série de données représentatives de la période respiratoire.

**16.** Dispositif selon la revendication 15, caractérisé en ce qu'il comprend des moyens (532, 534) d'analyse d'au moins une desdites séries de valeurs, effectuant au moins un des traitements appartenant au groupe comprenant :

- calcul (532) de moyennes et/ou de statistiques ;
- estimation et/ou prédiction (534) de valeurs futures ;
- génération d'alarme (536) en cas d'évolution alarmante.

**17.** Dispositif selon l'une quelconque des revendications 1 à 16, caractérisé en ce qu'il comprend des moyens (19 ; 527 ; 933) d'analyse d'au moins une desdites informations en fonction d'au moins une valeur de référence, de façon à fournir une information représentative d'un état (528) dudit sujet.

**18.** Dispositif selon la revendication 17, caractérisé en ce que lesdits moyens d'analyse comprennent des moyens (527) de reconnaissance d'au moins une phase du sommeil.

**19.** Dispositif selon la revendication 18, caractérisé en ce qu'il comprend des moyens de programmation d'un instant approximatif et d'une phase de sommeil, permettant d'assurer un réveil lors de cette phase de sommeil.

**20.** Dispositif selon l'une quelconque des revendications 1 à 19, caractérisé en ce qu'il comprend de plus au moins un des moyens appartenant au groupe comprenant :

- moyens de visualisation (529) ;
- horloge (543) ;
- moyens de réveil (536, 540) ;
- moyens de programmation (538) desdits moyens de réveil ;
- moyens de mémorisation (530) ;
- moyens de communication (537) avec une unité de traitement extérieur ;
- moyens de mesure de la température du corps et/ou du tonus musculaire dudit sujet.

**21.** Dispositif selon l'une quelconque des revendications 1 à 20, caractérisé en ce que l'ensemble des moyens le constituant sont réunis dans un boîtier portatif autonome (41).

**22.** Dispositif selon l'une quelconque des revendications 1 à 20, caractérisé en ce qu'il comprend d'une part un boîtier portatif autonome (41), comprenant au moins lesdits capteurs, et d'autre part des moyens de traitement distants (fig. 9), comprenant au moins une partie desdits moyens de traitement et d'analyse,
ledit boîtier comprenant de plus des moyens d'émission hertzienne et lesdits moyens de traitement distants comprenant de plus des moyens de réception, de façon à permettre la transmission des signaux relevés par lesdits capteurs.

**23.** Utilisation du dispositif selon l'une quelconque des revendications 1 à 22 pour au moins une des applications appartenant au groupe comprenant :

- reconnaissance des phases du sommeil ;
- réveil du sujet dans une phase de sommeil prédéterminée et/ou dans un intervalle de temps déterminé ;
- détection de la somnolence ;
- quantification du sommeil ;
- aide à la détection des troubles du sommeil ;
- aide à la détection des apnées ;
- aide à la surveillance de maladies cardio-vasculaires ;
- programmation d'appareils en fonction des phases du sommeil ;
- aide à l'analyse de la maladie de Parkinson ;
- aide à la prévention des ischémies cérébrales ;
- surveillance respiratoire ;
- surveillance de nourrisson ;
- analyse du sommeil polyphasique ;
- observation de la récupération après un effort ;
- suivi d'un niveau de vigilance ;
- aide à la détection du stress ;
- applications à l'élevage.

**Claims**

**1.** Device for the determination of physiological information, comprising:

- a first sensor (11; 21; 51) suitable for placing in contact with the skin of a subject and reacting to variations (12; 56) in the instantaneous blood pressure of the said subject, producing a representative signal (13; 53; 95) of the said instantaneous blood pressure,
- a second sensor (28; 55) remote from the skin of the said subject and reacting to the movements of the said subject, producing a signal (57; 94) representative of the said movements,

characterised in that it comprises:

- means (14; 59; 513; 912) for reducing interference present in the said representative signal (13; 53; 95) of the instantaneous blood pressure due to the said movements, as a function of the said representative signal (57; 94) of the said movements, said reducing means producing a corrected signal, and
- processing and analysing means (14; 515, 517, 546, 547, 548, 523; 98, 910, 913, 915, 919, 920, 921, 922, 923, 924, 927, 928, 929, 931) for the said corrected signal, producing at least one of the information elements belonging to the group comprising:

  - an information element (15; 518; 925) representative of the relative arterial pressure of the said subject and/or the change therein as a function of time;
  - an information element (112; 545; 932) representative of the respiration frequency of the said subject and/or the change therein as a function of time;
  - an information element (111: 545; 930) representative of the respiration amplitude of the said subject and/or the change therein as a function of time,

  and in that the said first sensor (11) comprises:
- a first piezo-electric element (21) reacting by flexion and/or traction/compression to the movements of a first sensor plate (24 (sic)) in direct contact with the body of the said subject and producing a first potential difference which is a function of the said movements of the first sensor plate (24 (sic)), and
- a second compensating piezo-electric element (27) reacting by flexion and/or traction/compression to the movements of a second sensor plate (28) remote from the body of the said subject and producing a second potential difference which is a function of the said movements of the second sensor plate (28).

2. Device according to Claim 1, characterised in that the said means for reducing interference comprise:

   - a differentiator (310; 59) provided, on the one hand, with the said representative signal (13; 311; 510) of the instantaneous blood pressure and, on the other hand, with the said representative signal (38; 511) of the said movements, producing a first corrected signal (39; 512; 950), and
   - means (513; 912) for adaptive digital filtering of the said first corrected signal, finding correlations between the said first corrected signal and the said representative signal of the said movements and suppressing, in the said first corrected signal, the elements corresponding to the said correlations, so as to produce a second digital corrected signal (514; 926).

3. Device according to either of Claims 1 and 2, characterised in that the said first and second piezo-electric elements (21, 27) are essentially identical and positioned in spaced overlay.

4. Device according to any one of Claims 1 to 3, characterised in that the said first and/or the said second piezo-electric elements (21, 27) are placed above and/or below an aperture (23) provided in a printed circuit (22) and fixed to the said printed circuit (22) by their ends (21$_A$, 21$_B$).

5. Device according to Claim 4, characterised in that the said first and/or the said second piezo-electric elements (21, 27) are glued to the said printed circuit (22) by their ends (21$_A$, 21$_B$) with the aid of a conducting glue.

6. Device according to any one of Claims 1 to 5 producing at least one information element (15; 518; 925) representative of the relative arterial pressure, characterised in that it comprises means (519) for determining the application pressure (45) of the said first sensor (21; 51) on the body of the said subject, producing a signal (18; 520; 96) representative of the said application pressure, and in that the said processing and analysing means (14; 517; 923) correct the said information element representative of the relative arterial pressure (15; 516) as a function of the said representative signal (18; 520; 96) of the application pressure.

7. Device according to Claim 6, characterised in that it comprises means (42) for positioning and/or fixing by clamping the said housing (41) in contact with the body of the said subject and in that the said measurement means (519) comprise a sensor reacting to the tension of the said positioning and/or clamping means (42).

8. Device according to any one of Claims 1 to 7, characterised in that the said processing and analysing means (14) comprise means (515, 517; 922, 923) for computing the relative arterial pressure (518) of the said subject, comprising at least one of the means belonging to the group comprising:

   - means (515; 922) for detecting the extremes (516) of the said signal (514) representative of the instantaneous blood pressure over a predetermined period of time,
   - checking and correction means (517) implementing a probability algorithm,

- means (517; 923) for correcting the relative arterial pressure as a function of the pressure exerted (520; 96) by the said first sensor on the body of the said subject.

9. Device according to any one of Claims 1 to 8, characterised in that it comprises means for determination of at least one of the information elements (17; 99, 911) representative of the actimetry of the said subject, belonging to the group comprising:

    - the average speed (911),
    - the average acceleration (99),
      starting from the said second signal representative of the movements.

10. Device according to any one of Claims 1 to 9, characterised in that it comprises means for determining at least one information element (111, 112; 545; 930, 932) representative of the respiration of the said subject, starting from an analysis (547, 548; 927, 928, 929, 931) of the maxima and/or the minima of the said signal representative of the said instantaneous blood pressure.

11. Device according to Claim 10, characterised in that it comprises at least one of the means belonging to the group comprising:

    - means (547; 927) for detecting the maxima and/or the minima of the said representative signal (13) of the said instantaneous blood pressure;
    - means (548; 928) for determination and suppression of the average value of the said maxima and minima, respectively;
    - means (548; 929) for computation of the absolute value of the signal formed by the said maxima and minima, respectively, representative of the respiration amplitude;
    - means (548; 931) for computation of the period of the signal formed by the said maxima and minima, respectively, representative of the respiration period.

12. Device according to any one of Claims 1 to 11, characterised in that it comprises means (523; 915) for computation of the cardiac rhythm (526; 916) of the said subject.

13. Device according to Claim 12, characterised in that the said means for computation of the cardiac rhythm comprise means (523) for determination of the period of the basic frequency of the said signal (514) representative of the instantaneous blood pressure.

14. Device according to Claim 13, characterised in that the said determination means comprise multichannel filtering means (523) comprising a plurality of filters ($524_1$ to $524_N$) each having a distinct frequency band, and means (525) for detecting the filter corresponding to the said basic frequency period.

15. Device according to any one of Claims 1 to 14, characterised in that it comprises memory means (530) for a series of relative arterial pressure values and/or a series of cardiac rhythm values and/or a series of values representative of the actimetry and/or a series of data representative of the respiration amplitude and/or a series of data representative of the respiration period.

16. Device according to Claim 15, characterised in that it comprises means (532, 534) for analysis of at least one of the said series of values, performing at least one of the processing operations belonging to the group comprising:

    - computation (532) of averages and/or statistics;
    - estimation and/or prediction (534) of future values;
    - generation of an alarm (536) in the event of a change giving cause for alarm.

17. Device according to any one of Claims 1 to 16, characterised in that it comprises means (19; 527; 933) for analysis of at least one of the said information elements as a function of at least one reference value, so as to provide an information element representative of a state (528) of the said subject.

18. Device according to Claim 17, characterised in that the said analysis means comprise means (527) for recognising at least one phase of sleep.

19. Device according to Claim 18, characterised in that it comprises means for programming an approximate instant and a phase of sleep, allowing waking during said phase of sleep to be ensured.

20. Device according to any one of Claims 1 to 19, characterised in that it also comprises at least one of the means belonging to the group comprising:

    - display means (529);
    - clock (543);
    - waking means (536; 540);
    - means for programming (538) the said waking means;
    - memory means (530);
    - means for communication (537) with an external processing unit;
    - means for determining the temperature of the body and/or the muscular tonus of the said subject.

21. Device according to any one of Claims 1 to 20, characterised in that all of the means making up said device are combined in an autonomous portable housing (41).

22. Device according to any one of Claims 1 to 20, characterised in that it comprises, on the one hand, an autonomous portable housing (41), comprising at least the said sensors, and, on the other hand, remote processing means (Fig. 9), comprising at least part of the said processing and analysing means, the said housing also comprising microwave transmission means and the said remote processing means also comprising receiving means, so as to allow transmission of the signals read by the said sensors.

23. Use of the device according to any one of Claims 1 to 22 for at least one of the applications belonging to the group comprising:

- recognition of phases of sleep;
- waking the subject in a predetermined phase of sleep and/or within a predetermined time interval;
- detection of somnolence;
- quantification of sleep;
- aid in detection of sleep disorders;
- aid in detection of apnoeas;
- aid in monitoring cardiovascular diseases;
- programming equipment as a function of the phases of sleep;
- aid in the analysis of Parkinson's disease;
- aid in the prevention of cerebral ischaemias;
- respiration monitoring;
- monitoring of infants not yet weaned;
- analysis of polyphase sleep;
- observation of recovery following effort;
- monitoring a vigilance level;
- aid in the detection of stress;
- applications in rearing.

**Patentansprüche**

1. Vorrichtung zum Ermitteln physiologischer Informationen, die über folgendes verfügt:

- einen ersten Fühler (11; 21; 51), der so ausgelegt ist, daß er in Berührung mit der Haut einer überwachten Person angebracht werden kann, und der auf die augenblicklichen Blutdruckvariationen (12;56) dieser Person reagiert und ein repräsentatives Signal (13; 53; 95) für den augenblicklichen Blutdruck liefert und
- einen zweiten Fühler (28; 55), der von der Haut der obigen Person isoliert ist, der auf deren Bewegungen reagiert und ein repräsentatives Signal (57; 94) dieser Bewegungen liefert, gekennzeichnet durch:

- Mittel (14; 59; 513; 912) zum Reduzieren von Störungen, die in dem für den augenblicklichen Blutdruck repräsentativen Signal (13; 53; 95) vorhanden sind und durch die Bewegungen verursacht werden, als Funktion des repräsentativen Signals (57; 94) dieser Bewegungen, wobei die Mittel zum Reduzieren ein berichtigtes Signal liefern und
- Mittel zum Verarbeiten und zum Analysieren (14; 515, 517, 546, 547, 548, 523; 98, 910, 913, 915, 919, 920, 921, 922, 923, 924, 927, 928, 929, 931) des berichtigten Signals, die mindestens eine der Informationen aus folgenden Gruppen liefert:
- eine für den relativen arteriellen Blutdruck der überwachten Person und/oder für die zeitliche Änderung dieses Blutdrucks repräsentative Information (15; 518; 925);
- eine für die Atemfrequenz der überwachten Person und/oder für die zeitliche Änderung dieser Atemfrequenz repräsentative Information (112; 545; 932);
- eine für die Atmungsamplitude der überwachten Person und/oder für die zeitliche Änderung dieser Atmungsamplitude repräsentative Information (111; 545; 930), und dadurch, daß der erste Fühler (11) folgendes umfaßt:
- ein erstes piezoelektrisches Element (21), das auf Biegung und/oder auf Zug/Druck auf die Bewegungen einer ersten Fühlerplatte (24) reagiert, welche unmittelbaren Kontakt zum Körper der überwachten Person hat und eine erste Spannungsdifferenz erzeugt, als Funktion der Bewegungen der ersten Fühlerplatte (24) und
- ein zweites piezoelektrisches Ausgleichselement (27), das auf Biegung und/oder auf Zug/Druck auf die Bewegungen einer zweiten Fühlerplatte (28) reagiert, welche vom Körper der überwachten Person isoliert ist und eine zweite Spannungsdifferenz erzeugt, als Funktion der Bewegungen der zweiten Fühlerplatte (28).

2. Vorrichtung gemäß Anspruch 1, dadurch gekennzeichnet, daß die Mittel zum Verringern der Störungen folgendes umfassen:

- eine Differenziervorrichtung (310; 59), in die einerseits das für den augenblicklichen Blutdruck repräsentative Signal (13; 311; 510) und andererseits das für die Bewegungen repräsentative Signal (38; 511) eingegeben wird und die ein erstes berichtigtes Signal (39; 512; 95) liefert, und

- Mittel (513; 912) zur anpassenden digitalen Filterung des ersten berichtigten Signals, welche Korrelationen zwischen dem ersten berichtigten Signal und dem für die Bewegungen repräsentativen Signal suchen und die im ersten berichtigten Signal die Elemente eliminieren, die den zuerst genannten Korrelationen entsprechen, um ein zweites berichtigtes digitales Signal (514; 926) zu liefern.

3. Vorrichtung gemäß einem der Ansprüche 1 oder 2, dadurch gekennzeichnet, daß das erste und das zweite piezoelektrische Element (21, 27) in etwa identisch und übereinander mit einem Zwischenraum angebracht sind.

4. Vorrichtung gemäß einem der Ansprüche 1 bis 3, dadurch gekennzeichnet, daß das erste und/oder das zweite piezoelektrische Element (21, 27) oberhalb und/oder unterhalb einer Aussparung (23) in einem gedruckten Schaltkreis(22) angebracht und über deren Enden (21$_A$, 21$_B$) an dem gedruckten Schaltkreis befestigt sind.

5. Vorrichtung gemäß Anspruch 4, dadurch gekennzeichnet, daß das erste und/oder das zweite piezoelektrische Element (21, 27) auf dem gedruckten Schaltkreis (22) über ihre Enden (21$_A$, 21$_B$) mit einem leitenden Kleber aufgeklebt sind.

6. Vorrichtung gemäß einem der Ansprüche 1 bis 5, die mindestens eine für den relativen arteriellen Blutdruck repräsentative Information (15; 518; 925) liefert, dadurch gekennzeichnet, daß sie über Mittel (519) zum Messen des Anpreßdrucks (45) des ersten Fühlers (21; 51) auf dem Körper der überwachten Person verfügt und ein für diesen Anpreßdruck repräsentatives Signal (18; 520; 96) liefert und, daß die Mittel zum Verarbeiten und Analysieren (14; 517; 923) die für den relativen arteriellen Blutdruck repräsentative Information (15; 516) als Funktion des für den Anpreßdruck repräsentativen Signals (18; 520; 96) berichtigen.

7. Vorrichtung gemäß Anspruch 6, dadurch gekennzeichnet, daß sie über Mittel (42) zum Positionieren und/oder zum Befestigen des Gehäuses (41) durch Andrücken an den Körper der überwachten Person verfügt und, daß die Mittel zum Messen (519) einen Fühler umfassen, der auf die Spannung der Positionierungs- und/oder Andruckmittel (42) reagiert.

8. Vorrichtung gemäß einem der Ansprüche 1 bis 7, dadurch gekennzeichnet, daß die Mittel zum Verarbeiten und Analysieren (14) Mittel (515, 517; 922, 923) zum Berechnen des relativen arteriellen Blutdrucks (518) der überwachten Person umfassen, welche mindestens ein Mittel aus der folgenden Gruppe enthalten:

- Mittel (515; 922) zum Erfassen der Extremwerte (516) des für den augenblicklichen Blutdruck repräsentativen Signals (514) während einer vorgegebenen Zeitdauer;
- Steuerungs- und Berichtigungsmittel (517), die einen Wahrscheinlichkeitsalgorithmus anwenden;
- Mittel (517; 923) zum Berichtigen des relativen arteriellen Blutdrucks als Funktion des vom ersten Fühler auf den Körper der überwachten Person ausgeübten Drucks (520; 96).

9. Vorrichtung gemäß einem der Ansprüche 1 bis 8, dadurch gekennzeichnet, daß sie über Mittel zum Feststellen von mindestens einer der für die Aktiometrie der überwachten Person repräsentativen Informationen (17; 99, 911) aus der folgenden Gruppe verfügt:

- die mittlere Geschwindigkeit (911),
- die mittlere Beschleunigung (99), ausgehend vom zweiten für die Bewegungen repräsentativen Signal.

10. Vorrichtung gemäß einem der Ansprüche 1 bis 9, dadurch gekennzeichnet, daß sie über Mittel zum Feststellen von mindestens einer für die Atmung der überwachten Person repräsentativen Information (111, 112; 545; 930, 932) verfügt, ausgehend von einer Analyse (547, 548; 927, 928, 929, 931) der Maxima und/oder der Minima des für den augenblicklichen Blutdruck repräsentativen Signals.

11. Vorrichtung gemäß Anspruch 10, dadurch gekennzeichnet, daß sie über mindestens eines der Mittel aus der folgenden Gruppe verfügt:

- Mittel (547; 927) zum Erfassen der Maxima und/oder der Minima des für den augenblicklichen Blutdruck repräsentativen Signals (13);
- Mittel (548; 928) zum Feststellen und zum Eliminieren des mittleren Wertes dieser Maxima bzw. Minima;
- Mittel (548; 929) zum Berechnen des Absolutwerts des von diesen Maxima bzw. Minima gebildeten Signals, welches für die Atmungsamplitude repräsentativ ist;
- Mittel (548; 931) zum Berechnen der Periode des durch diese Maxima bzw. Minima gebildeten Signals, welches für die Atmungsperiode repräsentativ ist.

12. Vorrichtung gemäß einem der Ansprüche 1 bis 11, dadurch gekennzeichnet, daß sie über Mittel (523; 915) zum Berechnen des Herzrhythmus (526; 916) der überwachten Person verfügt.

13. Vorrichtung gemäß Anspruch 12, dadurch gekennzeichnet, daß die Mittel zum Berechnen des Herzrhythmus Mittel (523) zum Feststellen der Periode der Grundschwingung dieses für den augenblicklichen Blutdruck repräsentativen Signals (514) umfassen.

14. Vorrichtung gemäß Anspruch 13, dadurch gekennzeichnet, daß die Feststellungsmittel Mehrkanal-Filtermittel (523) umfassen, unter denen sich eine Vielzahl von Filtern ($524_1$ bis $524_N$) befinden, die jeweils ein anderes Frequenzband haben, und Mittel (525) zum Bestimmen des der Periode der Grundschwingung entsprechenden Filters enthalten.

15. Vorrichtung gemäß einem der Ansprüche 1 bis 14, dadurch gekennzeichnet, daß sie über Mittel (530) verfügt zum Speichern einer Reihe von Werten des relativen arteriellen Drucks und/oder einer Reihe von Werten des Herzrhythmus und/oder einer Reihe von Werten, die für die Aktiometrie repräsentativ sind und/oder einer Reihe von Werten, die für die Atmungsamplitude repräsentativ sind und/oder einer Reihe von Werten, die für die Atmungsperiode repräsentativ sind.

16. Vorrichtung gemäß Anspruch 15, dadurch gekennzeichnet, daR sie über Mittel (532, 534) zum Analysieren von mindestens einer der obigen Wertreihen verfügt, wobei mindestens eine der Verarbeitungen aus der folgenden Gruppe durchgeführt wird:

- Berechnung (532) von Mittelwerten und/oder von Statistiken;
- Schätzung und/oder Vorhersage (534) zukünftiger Werte;
- Erzeugung einer Alarmmeldung (536) im Falle einer besorgniserregenden Entwicklung.

17. Vorrichtung gemäß einem der Ansprüche 1 bis 16, dadurch gekennzeichnet, daß sie über Mittel (19; 527; 933) zum Analysieren von mindestens einer der obigen Informationen verfügt, als Funktion von mindestens einem Referenzwert, um eine für einen Zustand (528) der überwachten Person repräsentative Information zu liefern.

18. Vorrichtung gemäß Anspruch 17, dadurch gekennzeichnet, daß die Mittel zum Analysieren Mittel (527) zum Erkennen von mindestens einer Schlafphase umfassen.

19. Vorrichtung gemäß Anspruch 18, dadurch gekennzeichnet, daß sie über Mittel zum Programmieren eines approximierenden Augenblicks und einer Schlafphase verfügt, die ein Aufwachen während der Schlafphase sicherstellen.

20. Vorrichtung gemäß einem der Ansprüche 1 bis 19, dadurch gekennzeichnet, daß sie zusätzlich über eines der Mittel aus der folgenden Gruppe verfügt:

- Anzeigemittel (529);
- Uhr (543);
- Mittel zum Wecken (536, 540);
- Mittel zum Programmieren (538) der Mittel zum Wecken;
- Speichermittel (530);
- Mittel zum Kommunizieren (537) mit einer außenstehenden Verarbeitungsanlage;
- Mittel zum Messen der Körpertemperatur und/oder der Muskelanspannung der überwachten Person.

21. Vorrichtung gemäß einem der Ansprüche 1 bis 20, dadurch gekennzeichnet, daß die Menge der Mittel aus denen sie zusammengesetzt ist, in einem eigenen tragbaren Gehäuse (41) untergebracht sind.

22. Vorrichtung gemäß einem der Ansprüche 1 bis 20, dadurch gekennzeichnet, daß sie einerseits Ober ein eigenes tragbares Gehäuse (41) verfügt, das mindestens die Fühler umfaßt und andererseits über Mittel zur Fernbearbeitung (Fig. 9), die mindestens einen Teil der Analyse- und Verarbeitungsmittel umfassen, wobei das Gehäuse darüber hinaus Ober Funkmittel verfügt und wobei die Mittel zur Fernverarbeitung über Empfangsmittel verfügen, um das Übermitteln der von den Fühlern aufgenommenen Signale zu ermöglichen.

23. Anwendung der Vorrichtung gemäß einem der Ansprüche 1 bis 22 für mindestens eine der Anwendungen aus der folgenden Gruppe:

- Erkennung der Schlafphasen;
- Wecken der überwachten Person in einer vorgegebenen Schlafphase und/oder innerhalb eines festgelegten Zeitintervalls;
- Feststellen der Schläfrigkeit;
- Quantifizierung des Schlafs;
- Hilfe bei der Feststellung von Schlafstörungen;
- Hilfe bei der Erfassung von Apnöen (Atmungsaussetzern);
- Hilfe bei der Überwachung von Herz-Kreislauferkrankungen;
- Programmierung von Apparaten als Funktion der Schlafphasen;
- Hilfe bei der Analyse der Parkinsonkrankheit;
- Hilfe bei der Vorbeugung von Durchblutungsstörungen des Gehirns;
- Überwachung der Atmung;
- Überwachung von Säuglingen;
- Analyse des Mehrphasenschlafs;
- Beobachtung der Erholung nach einer Anstrengung;
- Verfolgung eines Überwachungsniveaus;
- Hilfe bei der Erfassung von Streß;
- Anwendungen beim Aufwachsen.

Fig. 1

Fig. 2

Fig. 3

Mouvement

Serrage

Pression + Mouvement

Fig. 4

Pression

SBP

MBP

DBP

t°

Fig. 6

Fig. 5

EP 0 681 447 B1

Fig. 7

Fig. 8

Fig. 9